(19) Europäisches Patentamt
European Patent Office
Office européen des brevets

(11) EP 4 216 980 B1

(12) EUROPEAN PATENT SPECIFICATION

(45) Date of publication and mention of the grant of the patent:
**10.04.2024 Bulletin 2024/15**

(21) Application number: **21777801.8**

(22) Date of filing: **21.09.2021**

(51) International Patent Classification (IPC):
***A61K 35/74*** *(2015.01)* ***A61K 38/16*** *(2006.01)*
***A61P 25/02*** *(2006.01)*

(52) Cooperative Patent Classification (CPC):
**A61K 35/74; A61K 38/168; A61P 25/02**

(86) International application number:
**PCT/EP2021/075902**

(87) International publication number:
**WO 2022/063761 (31.03.2022 Gazette 2022/13)**

(54) **C-PHYCOCYANIN FOR USE IN THE TREATMENT AND/OR PREVENTION OF PERIPHERAL NEUROPATHY**

C-PHYCOCYANIN ZUR VERWENDUNG BEI DER BEHANDLUNG UND/ODER PRÄVENTION VON PERIPHERER NEUROPATHIE

C-PHYCOCYANINE DESTINÉE À ÊTRE UTILISÉE DANS LE TRAITEMENT ET/OU LA PRÉVENTION DE LA NEUROPATHIE PÉRIPHÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.09.2020 EP 20382835**

(43) Date of publication of application:
**02.08.2023 Bulletin 2023/31**

(73) Proprietor: **Anilur Pharma, S.L.**
**08195 Sant Cugat del Valles Barcelona (ES)**

(72) Inventors:
- **SAN GABRIEL ALCOLEA, Consuelo**
  **08195 SAN CUGAT DEL VALLÉS (ES)**
- **GASCÓN VILAPLANA, Pere**
  **08036 BARCELONA (ES)**
- **BLUM, Emmanuel**
  **34160 SAINT-DRÉZÉRY (FR)**

(74) Representative: **ZBM Patents - Zea, Barlocci & Markvardsen**
**Rambla Catalunya, 123**
**08008 Barcelona (ES)**

(56) References cited:
**EP-A1- 3 192 504** **WO-A1-2021/099453**
**US-A1- 2012 258 180** **US-A1- 2014 287 021**

- **KIM YE-RI ET AL: "C-phycocyanin from Limnothrix Species KNUA002 Alleviates Cisplatin-Induced Ototoxicity by Blocking the Mitochondrial Apoptotic Pathway in Auditory Cells", MARINE DRUGS,, vol. 17, no. 4, 31 March 2019 (2019-03-31) , XP009525943, ISSN: 1660-3397, DOI: 10.3390/MD17040235**
- **IYYAPU KRISHNA MOHAN ET AL: "Protection against cisplatin-induced nephrotoxicity by Spirulina in rats", CANCER CHEMOTHERAPY AND PHARMACOLOGY, SPRINGER, BERLIN, DE, vol. 58, no. 6, 22 March 2006 (2006-03-22) , pages 802-808, XP019423345, ISSN: 1432-0843, DOI: 10.1007/S00280-006-0231-8**
- **QIAN LIU ET AL: "Medical Application of Spirulina platensis Derived C-Phycocyanin", EVIDENCE-BASED COMPLEMENTARY AND ALTERNATIVE MEDICINE, vol. 2016, 1 January 2016 (2016-01-01), pages 1-14, XP055670663, US ISSN: 1741-427X, DOI: 10.1155/2016/7803846**
- **KEFAYAT AMIRHOSEIN ET AL: "Abstract", SCIENTIFIC REPORTS , vol. 9, no. 1 1 December 2019 (2019-12-01), XP055780579, DOI: 10.1038/s41598-019-55605-w Retrieved from the Internet: URL:http://www.nature.com/articles/s41598-019-55605-w**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **VILLA FRANCISCO A. ET AL: "Marine natural product drug discovery: Leads for treatment of inflammation, cancer, infections, and neurological disorders", IMMUNOPHARMACOLOGY AND IMMUNOTOXICOLOGY, vol. 32, no. 2, 22 June 2010 (2010-06-22), pages 228-237, XP055859498, US ISSN: 0892-3973, DOI: 10.3109/08923970903296136 Retrieved from the Internet: URL:http://dx.doi.org/10.3109/08923970903296136>**
- **KHDOUR MAHER R: "Treatment of diabetic peripheral neuropathy: a review", JOURNAL OF PHARMACY AND PHARMACOLOGY : JPP, vol. 72, no. 7, 8 June 2020 (2020-06-08), pages 863-872, XP055859509, GB ISSN: 0022-3573, DOI: 10.1111/jphp.13241**
- **ABDEL-DAIM MOHAMED M ET AL: "Oral Spirulina Platensis Attenuates Hyperglycemia and Exhibits Antinociceptive Effect in Streptozotocin-Induced Diabetic Neuropathy Rat Model", JOURNAL OF PAIN RESEARCH, vol. Volume 13, 1 January 2020 (2020-01-01), pages 2289-2296, XP055862128, GB ISSN: 1178-7090, DOI: 10.2147/JPR.S267347**

## Description

This application claims the benefit of the European Patent Application no. 20382835.5 filed on September 22nd, 2020.

## FIELD OF THE INVENTION

The present invention relates to C-phycocyanin for use in the treatment and/or prevention of peripheral neuropathy.

## BACKGROUND ART

Peripheral Neuropathy (PN) is caused by a disorder which provides damage directly on neural tissues or affects neural tissues. The origin (causes) and the mechanism that can cause the PN can be very varied, but all PN share a common patho-physiology that involves extensive generation of reactive oxygen species (ROS), mitochondrial damage, axon degeneration, demyelination (loss of myelin sheath), and altered metabolic pathways, among others. They render mitochondrial dysfunction, oxidative stress, and neuroinflammation.

Depending on the type of neural tissues that are affected, peripheral neuropathy can be categorized into sensory neuropathy, motor neuropathy, and autonomic neuropathy. It usually starts with alterations in sensory perceptions and may be followed by visceral dysfunctions or impaired motility. In case of sensory neuropathy, a patient may feel numbness to touch, shaking, a reduced ability to sense temperature change, tingling feeling, burning pain, and also skin allodynia or the like. Meanwhile, motor neuropathy is accompanied with impaired balance, weakness of muscle strength, and a patient with autonomic neuropathy experiences, depending on an organ affected by corresponding neuron, urinary incontinence due to weakened bladder controlling activity, or has abnormal blood pressure and heartbeat.

Symptoms may range from mild to severe. The acute symptoms of PN can appear during treatments, or after they have already finished, being worse during active treatments. The acute symptoms of PN may become chronic and persist during months and years or even becoming lifelong. Thus, the quality of life of those patients suffering from PN can thus be considerably impaired, and in many cases, permanently diminished because of them. It is known in the state of the art diseases and conditions that (regardless the origin and mechanism) involve neurotoxic damages (PN) as a result of a chronic or acute inflammation, increased oxidative stress, and/or mitochondrial dysfunction/damage. Some of these diseases or conditions that cause PN are the following: diabetes, atherosclerosis, cancer, and the chemo- and radioanti-cancer therapy, infections, antibiotic therapy, antiretroviral therapy, atherosclerosis, chronic alcohol consumption, lung and kidney injuries and (non-alcohol) steatohepatitis.

Chemotherapy is one of the commonly condition that induces peripheral neuropathy, also called Chemotherapy-induced peripheral neuropathy (CIPN). It is known that the anti-cancer agent administered to a patient is accumulated not only in tumour tissues but also in peripheral nervous system where it exerts neuronal toxicity and induces CIPN. In fact, cytotoxic agents, most commonly referred to as cancer chemotherapy, are directed against tumour cells, seeking to bring about their eradication. They are one of the pillars of cancer treatments and they are routinely administered to an average of 70% of cancer patients, alone or in diverse combinations in different chemotherapy regimens. However, the cytotoxic effects of these molecules go well beyond their intended target and affect all actively dividing cells. Besides tumour cells, they simultaneously affect tissues that are particularly vulnerable to the various activities of anti-neoplastic agents such as neurons. They induce cell death by promoting inflammation, damaging mitochondria, triggering the massive production of reactive oxygen species (ROS), and causing severe oxidative stress. These activities inflicted on tumour and non-tumour cells lie at the basis of the undesirable effects of CIPN in a range from 11% to 87% of the patients.

Thanks to modern therapies, many patients survive cancer; however, they often suffer from acute toxicities which cause debilitating symptoms that may become chronic and persist long after treatments have been completed, or even lifelong. The symptoms related to CIPN include numbness in fingers and toes, tingling or burning feeling, cold feeling, pain, and weakened touch sense and muscle strength (T. Armstrong et al., 2005, Oncol Nurs Forum, v32, pp305-311; C. Visovsky et al., 2008, Clin J Oncol Nurs, v12, pp243-247). As it is mentioned above, the quality of life of those patients can thus be considerably impaired, and in many cases, permanently diminished because of them. In fact, CIPN is one of the most common and crippling acute or chronic consequences in cancer survivors.

Furthermore, CIPN may also reduce the dosage of the anti-cancer agent administered to a patient depending on severity of symptoms or, in an extreme case, may early terminate the anti-cancer therapy, which negatively affect anti-cancer therapy. It is known that about one third of patients receiving anticancer therapy are affected by CIPN, and one third of the patients with CIPN have a permanent neuronal damage (A. Bhagra and R.D. Rao, 2007, Curr Oncol Rep, v9, pp290-299).

Anti-cancer agents which are known to cause CIPN include platinum-based anticancer agents (like cisplatin and oxaliplatin), taxane-based anti-cancer agents (like paclitaxel and docetaxel), vinca alkaloids (like vincristine and

vinblastine), bortezomib, thalidomide and its analogs (lenalilomide), or the like. The incidence of CIPN depends not only on types, dosages, and administration periods of an anti-cancer agents (G. Cavaletti et al., 2011, Curr Treat Options Neurol, v13, pp180-190), but also on the previous health conditions and genetic factors of individual patients. Chemotherapy combinations with higher prevalence of CIPN include those that involve platinum drugs, vinca alkaloids, bortezomib, and/or taxanes. Particularly, the prevalence of CIPN is highest with platinum-based agents (oxaliplatin: 70 to 100%), taxanes (11 to 87%), thalidomide and related molecules (20 to 60%), and bortezomib 36%.

Taxane chemotherapeutic agents have been used to treat subjects with breast, ovarian, lung, bladder, and oesophageal cancer, among others. Representatives of taxanes include paclitaxel, including without limitation, e.g., Taxol™, Abraxane™, and the like, and analogs thereof, including, without limitation, polyglutamylated forms of paclitaxel (Xyotax™), liposomal paclitaxel (Tocosol™), and docetaxel and analogs and formulations thereof. However, the administration of taxanes, for example, is commonly limited due to the development of serious and potentially life-threatening toxicities. In particular, the clinical use of taxanes frequently involves delay, modification, or discontinuance of use due to chemotherapyassociated toxicities including toxic disorders of peripheral nerve systems (including CIPN) resulting in numbness, burning, pain, paraesthesia, dysesthesias, sensory loss, weakness, paralysis, arthralgia, and myalgia. CIPN associated to taxane drug treatment ranges from 11 to 87%. Paclitaxel is associated with the highest rates and causes more intense neuropathic symptoms than docetaxel. It affects mostly small fibres, producing paraesthesia, dysesthesias, loss of fine coordination, anaesthesia, and pain, predominantly in a glove/stocking distribution. Platinum analogue chemotherapeutic agents have been used to treat subjects with lung, head, neck, ovary, esophagus, bladder, testis, and other cancers. Representatives of platinum analogue chemotherapeutic agents include cisplatin, carboplatin, oxaliplatin and satraplatin. In the particular case of oxaliplatin, it is commonly used for the treatment of metastatic colorectal, choleangiocarcinoma, pancreatic, liver, oesophageal and stomach cancer, cancers with high incidence, particularly colorectal. Like the taxanes and other chemotherapeutic drugs, platinum analogs are associated with neurotoxicity and particularly with CIPN. Oxaliplatin induces a wide range of alterations in mitochondria and other organelles, membrane receptors, ion channels, neuron signalling pathways, and neurotransmission, which, as a whole, result in neuroinflammation, DNA damage and axonal degeneration. Oxaliplatin spontaneously produces reactive platinum species releasing oxalate in the process, which may contribute to the cold induced neuropathy. In fact, cold induced neuropathy is characteristic of the acute toxicity of oxaliplatin in hands and feet, often associated with pharyngolaryngeal dysphasia, spasms in the jaw and other striated muscles. The incidence of acute CIPN by oxaliplatin is very high, from 65% to 98% of patients develop different grades of it during active treatments. Chronic neuropathy can persist for more than two years in up to 84% of patients, in a hand/glove distribution. The intensity and extension of the acute forms are a risk factor for chronic forms. On the other hand, cisplatin is presently seldom included in chemotherapy regimens. The incidence of acute CIPN by cisplatin is also very high, from 49% to 100% of patients develop CIPN in different grades active treatments. Furthermore, besides CIPN, cisplatin can also cause ototoxicity, myelotoxicity, and nephrotoxicity. And, finally, the incidence of carboplatin, another member of the platinum salts family, is from 13% to 42% of treated patients.

Vinca alkaloids interfere with tubulin polymerization, altering axonal transport and producing distal axonopathy. They also attract immune cells that mediate neuroinflammation, and damage myelin sheaths. Approximately 40% of patients treated with this class of anti-cancer drugs develop CIPN and particularly those treated with vincristine which is the most neurotoxic of the vinca alkaloids (used in haematological cancers -plastic leukaemia).

Bortezomib is a reversible proteasome inhibitor that is administered in the treatment of multiple myeloma and certain types of lymphoma. It causes painful sensory neuropathy and demyelinising neuropathy, predominantly in a chronic fashion, in 34% of patients. Symptoms may persist for months or years after completion of chemotherapy. Bortezomib causes CIPN through three mechanisms: enhancement of sphingolipid metabolism in astrocytes, mitochondrial damage, and attraction and activation of T-lymphocytes. These activities result in neuropathic pain through release of glutamate, production of ROS with subsequent oxidative stress, and neuroinflammation.

Thalidomide and its analogue lenalilomide are used for the treatment of multiple myeloma. They inhibit angiogenesis, modify the synthesis of NF-kB and TNF, and generate a dihydroxy metabolite that triggers extensive release of ROS, inducing neuropathy (CIPN) in 25 to 75% of patients.

Therefore, the treatment of cancer with all the anti-cancer active ingredients mentioned above (platinum-based and taxane-based anticancer agents, vinca alkaloids, bortezomib and thalidomide and its analogues) regardless the causes and the mechanism, cause in a minor or major extension toxicity in the peripheral nervous system inducing peripheral neuropathy.

Diabetes is also a disease that induces peripheral neuropathy, also called Diabetes induced peripheral neuropathy (DPN). In fact, diabetes has become an epidemic problem worldwide, 366 million people are predicted to suffer from the disease in 2030, doubling the numbers of 2000. Further, peripheral neuropathy is the most prevalent complication of diabetes, and is the most difficult to treat. DPN is the main cause of polyneuropathy in the United States US, affecting up to 70% of diabetic patients, in patients diagnosed since more than 25 years, but up to 90% if subclinical manifestations are considered. In this case, sensory, motor and autonomic nerves may be affected, usually presenting a symmetric pattern in a stocking distribution, and affecting mobility, which increases the risk of falls, ulcers, and may cause severe,

chronic neuropathic pain, interfering with sleep, and linked to anxiety and depression. Loss of sensory perception in the lower limbs is also a risk factor for limb amputation, which may affect up to 2% of patients. It is known in the state of the art that chronically high glucose levels lead to metabolic cascades that end in nerve injury. Vascular and anatomical characteristics of peripheral nerves, make them particularly vulnerable to ischemia and subsequent tissue damage. Vascular autonomic nerves regulating blood flow through sparse transperineural arterioles, fundamental for the supply of peripheral nerves, are constituted by cells whose bodies lie in sympathetic nerve ganglions. Its extensively long axons, covered by Schwann cells, are too weak to support themselves for the long transport of nutrients, nerve trophic factors and other signals. In addition, the levels of blood flow are not autoregulated. Nerve terminals on the distal side are not covered by the perineum and more exposed to traumatic injuries. Glycation end products trigger NADP oxidase and the production of ROS with the associated oxidative stress. Free radical production, with defective antioxidant anti-oxidant defence pathways generating uncontrolled oxidative stress, has been linked to DPN. This last is considered as the main factor causing DPN, together with a proinflammatory environment. There is evidence that diabetic nerves are susceptible to ischemia/reperfusion injury a proinflammatory process.

As it is mentioned above, infections caused by virus, bacteria and/or parasite can also cause peripheral neuropathy.

Regarding virus induced peripheral neuropathy, on one hand, in viral infections caused by Human immune deficiency virus (HIV), Human T-cell lymphotropic virus (HTLV), Herpes simplex (HSV 1 and 2), Varicella zoster Virus (VZV), Cytomegalovirus (CMV), Epstein-Barr virus (EBV), West Nile virus (WNV), Hepatitis C virus (HCV) and Rabies virus (RV) have been well described. Some bacterial infections are also at the origin of peripheral neuropathies. They can inflict direct damage to nerve endings, such as *Mycobacterium leprae* or *Mycobacterium tuberculosis,* or indirectly through the production of neurotoxins like *Clostridium botulinum,* or *Corynebacterium diphteriae* (23). Furthermore, not only the infections but also numerous therapeutic agents used to treat the infectious diseases can also be responsible of length dependent small-fiber neuropathies. Fluoroquinolones, isoniazid, ethambutol and aminoglycosides are well recognized neurotoxic antibiotics. Dideoxinucleoside antiretrovirals used for HIV patients may also cause a neuropathic syndrome in long term treatments. It is disclosed in the state of the art that Varicella zoster virus (VZV) is responsible for two distinc human diseases: varicella (chicken pox), that results from the initial infection with the virus during the childhood or adolescent years, and herpes zoster, due to the reactivation of the infection later in life. VZV can remain for years and decades in a dormant state in cranial nerves and dorsal ganglia, and risk of reactivation is related to old age, poor immune competence, or early age of chicken pox infection. About 90% of people that have suffered from initial varicella are at risk of reactivation at during their lifetime. Herpes zoster recurrence is rare, only seen in 5% of patients, probably thanks to a robust immune response elicited by the VZV reactivation causing the herpes zoster disease. Approximately 2 over 1000 persons per year will suffer from shingles, up to 12/1000 if older than 65. About 10% to 20% of immunocompetent adults will develop PHN. It is usually accompanied by a painful dermatome (typical skin lesions starting with a rash, that evolves into blisters and final crusts), even though, in some cases, the dermatome can be absent (*zoster sine herpete,* or zoster without herpes). Pain and neuropathy symptoms may range from absent in children to excruciating, and they can last for days, weeks, years or for a lifetime. PHN is the most common complication of herpes zoster. It is defined as a significant pain or abnormal sensory perceptions that lasts 120 days or more after the apparition of the initial rash, and after its complete resolution. Its incidence varies between 10% and 20% in immune competent adults, age being the main risk factor. Incidence ranges from 2% for those younger than 50 but reaches 35% over the age of 85. This number correlate with the severity and extension of the dermatomal eruptions, that reflect a greater nerve damage. PHN greatly interferes with the normalcy in the life of patients. It alters sleep patterns, ability to concentrate and pursue a professional activity, causes chronic fatigue, depression, anxiety, anorexia, and social avoidance. Regarding the mechanism of action, peripheral neuronal injuries are directly caused by the VZV. Yet, PHN symptoms of pain, dysesthesias, and paraesthesia involve central and peripheral structures of the nervous system. Damage causes neurons to generate spontaneous discharges from edge excitable loci triggering pain sensations with light stimuli. As with DPN, epidermal free nerve endings appear greatly reduced or lost, causing alterations in sensory perception signals. And as with CIPN, voltage gated sodium channels are altered and overexpressed, causing damaged areas of the axons to send hyperalgesia signals in the absence of proportional triggers. Central sensitization is developed by the intensified excitability of the spinal cord neuron as a response to the repeated signals of peripheral injury.

On the other hand, virus induced peripheral neuropathy caused by coronavirus have been also described. Deadly coronavirus infections include Severe acute respiratory syndrome (SARS), a viral respiratory disease of zoonotic origin caused by severe acute respiratory syndrome coronavirus (SARS-CoV or SARS-CoV-1); Middle East respiratory syndrome (MERS), a viral respiratory infection caused by the MERS-coronavirus (MERS-CoV); and Coronavirus disease 2019 (COVID-19), an infectious disease caused by the severe acute respiratory syndrome coronavirus 2 (SARS-CoV-2). SARS-CoV-2 is a positive-sense single-stranded RNA virus. In humans, coronaviruses mainly cause respiratory tract infections and gastrointestinal manifestations than can be lethal. Yet, multiorgan manifestations are common and caused by a combination of different mechanisms which include direct viral toxicity, microvascular injury, immune system dysregulation with a hyperinflammatory state, and hypercoagulability. Neurological complications have been increasingly

recognised in a number of patients, 38% of them suffered from persistent neurological alterations 6 weeks after infection. About 10% of patients experience a loss of taste and smell up to 6 months of follow up. Studies show that cognitive impairment, brain fog, memory losses, dysautonomia, and alterations in language reception occur in 20% to 40% of patients discharged from ICU. The pathophysiology appears to involve direct viral damage and neuroinflammation, microvascular thrombosis and neurodegeneration. Viral invasion of the extracellular spaces of the olfactory epithelium and chronic neuronal injury have been found in patients with more severe infections. Thus, as in other related coronavirus infections, neuronal damage has been a common manifestation in COVID-19 disease. The pathophysiological mechanism seems to involve direct viral damage and neuroinflammation, as is the case with other viral related peripheral neuropathies.

Further, chronic alcohol consumption is also one of the causes of peripheral neuropathy, the mechanisms are common to other peripheral neuropathy (for instance, chronic or acute inflammation, increased oxidative stress, and/or mitochondrial dysfunction/damage), with added particularities such as thiamine deficiency and multi system damage, including central nervous system (CNS) structures.

The, as it is disclosed in the state of the art, the pathophysiology of the peripheral neuropathy caused by these diverse conditions has common features. Acute or chronic inflammation, oxidative stress, loss of the myelin sheath and mitochondrial dysfunctions affecting peripheral neurons are the shared pathophysiological mechanisms that constitute the damage.

The locations, intensity and extension of the symptoms of peripheral polyneuropathy depend on the sites, amplitude, and degree of the nerve damage. Pharmacological treatments have been disclosed in the state of the art for the treatment of peripheral neuropathy. A number of drugs are administered to prevent, treat or alleviate the pain and associated nerve disfunctions with limited success. in particular, palliative treatments address to reduce or eliminate the symptoms have been disclosed. Anti-seizure agents like gabapentin or anti-depressants like amitriptyline are clinically used to alleviate CIPN-related symptoms, and particularly to ameliorate the neuropathic pain. However, they are not addressed to solve the causes of pain and the remaining neuropathic symptoms. In fact, large scale clinical studies have failed to prove their efficacy for CIPN treatment. Furthermore, those drugs basically exhibit adverse side effects like dizziness, sleepiness, or the like, and have a disadvantage regarding to its dependence and addiction effect (for opioid derivative such as fentanyl), as well as that administration at high dose is impossible due to their low safety margin.

A second strategy for the treatment chemotherapy induced peripheral neuropathies (CIPN) has been disclosed in the state of the art which involves the use of active ingredients that treat the causes, but individually (unitargeted treatment) and mainly the neuroinflammation. In particular, the United States patent application US2014287021 patent application discloses the use of thiosemicarbazone compounds. Further, the European patent application EP3192504 discloses the use of IL-8 inhibitor compounds, and preferably dual CXCR1/CXCR2 receptor inhibitors. Meanwhile, in the United States patent application US201258180 it is disclosed the use of poly(ADP-ribose)polymerase (PARP) inhibitor. However, none of them are capable of treating all the causes at the same time and being effective enough for being used in the treatment of chemotherapy-induced peripheral neuropathy. In fact, nowadays, there is no standard therapeutic agent available for the treatment of prevention of chemotherapy induced peripheral neurotherapy, and even less a drug approved by a Regulatory Drug Agency (such as EMA or FDA) for CIPN treatment.

Presently, the standard of care for CIPN includes dose reduction and/or discontinuation of chemotherapy treatment but this is, obviously, to the detriment of tumour growth control. Such medical unmet needs make it urgent to develop a novel safer and more effective therapeutic or preventive agent of CIPN. Because CIPN is considered a complex disease, a multi-target-based drug development may be more appropriate, and thus medicinal plants or natural products which have been traditionally used can be important starting materials from the viewpoint of drug development strategy. Various drugs and nutraceuticals such as vitamin E, acetyl-L-carnitine, glutamine, glutathione, vitamin B6, omega-3 fatty acids, magnesium, calcium, alpha lipoic acid, and N-acetyl cysteine have been proposed for use as adjuvants during chemotherapy to reduce CIPN but a review from Schloss J.M. et al., Clin Nutr (2013) 32(6), pp 888-893 concludes that none of them has shown solid beneficial evidence to be recommended for the treatment and/or prophylaxis of CIPN. This conclusion is also supported by Hershman D.L. et al., J Clin Oncol (2014) 32(18), pp 1941-1967 where the authors conclude that there are no established agents recommended for the prevention of CIPN in patients with cancer undergoing treatment with neurotoxic agents. As with chemotherapy induced peripheral neuropathies, oral drugs like GABA analogues (Pregabalin and Gabapentin), tricyclic or inhibitors of serotonin recapture antidepressants (like Duloxetine), or opioids (like Tramadol) may benefit some patients suffering from diabetes or infection induced peripheral neuropathy. Further, local administration with capsaicin or transdermal lidocaine are also recommended as third line therapies. However, none of these treatments address the underlying mechanisms that cause pain and they are palliative in their purpose. Only moderate pain relief is achieved through these pharmacological approaches.

Thus, there exists an unmet need to find a product which treat and/or prevent the symptomatology of peripheral neuropathy (PN), and particularly the chemotherapy-induced peripheral neuropathy (CIPN). Therefore, from what is known in the state of the art, there is still the need of providing an effective and safe (non-toxic) active ingredient useful for the prophylaxis and/or treatment of peripheral neuropathy, and particularly the chemo-induced peripheral neuropathy.

## SUMMARY OF THE INVENTION

The invention is defined in claims 1-15.

The inventors have now successfully and surprisingly found that C-phytocyanin is useful for the treatment and/or prevention of peripheral neuropathy. As it is demonstrated in the experimental section, the inventors have found that C-phytocyanin is useful for the treatment and/or prevention of peripheral neuropathy regardless the origin (causes) and mechanism that causes the neurotoxicity. Particularly, the administration of C-phytocyanin is capable of preventing and/or treating the PN caused by chemotherapeutic treatment (CIPN), diabetes, and infections such as viral (herpes virus and coronavirus as COVID-19 disease), bacterial or parasitic infections, which involves chronic or acute inflammation, increased oxidative stress, and/or mitochondrial dysfunction/damage.

Without being bound to any theory, it seems that the C-phycocyanin is a multi-target nutraceutical compound having simultaneous activity as mitochondrial protector, antioxidant (highly effective ROS scavenger and NADPH inhibitor), anti-inflammatory (COX-2 inhibitor) and myelin protector that contribute in a combined (synergic) manner to its neuronal therapeutic and preventive properties, unlike the treatments disclosed in the state of the art that either address one cause, or are only symptomatic treatments. It is advantageous because allows improving patient's quality of life.

Particularly, the inventors have surprisingly found that the administration of C-phycocyanin to patients who are receiving chemotherapy or have received chemotherapy for the treatment of cancer and further suffer or are susceptible of suffering from chemotherapy-induced peripheral neuropathy (CIPN) is capable of ameliorating or eliminate said neuropathy. As it is shown in the experimental section, the C-phytocyanin is useful for preventing, ameliorating or completely eliminating (treat) the neuropathy induced by the most commonly used anti-cancer agents, for instance, platinum-based agents, taxane-based agents, thalidomide and analog thereof, bortezomib and alkylated agents (such as capecitabin). In fact, C-phycocyanin was efficient in completely correcting CIPN in 60% of patients or reducing it to mild symptoms in 40% of cases, mainly limited in these to slight sensorial alterations in toes, in about 4 to 8 weeks. It is advantageous because allows improving patient's quality of life during treatments, observance, efficacy of chemotherapy, and patient's outcomes.

To sum up, the present invention discloses methods, as well as compositions to achieve higher degrees of patient safety by reducing chemotherapy-induced peripheral neuropathy (CIPN). By significantly increasing the degree of safety of the patient's overall treatment with chemotherapy and reducing adverse physiological responses to chemotherapeutic pharmacological intervention, the methods, compositions, and formulations of this invention may, for example:

(i) allow physicians to keep with the programed schedule in terms of number of cycles and also without reducing the doses of the chemotherapy agent, by keeping them this has been demonstrated to improve the outcome of the patients

(ii) allows physicians to avoid delay in the treatments of discontinuations of the treatments, by keeping "them" to optimize the outcome of the patients due to the PN a large amount of patients have discontinuation of the treatments,

(iii) allow increases in the number of chemotherapy treatments by the prevention of cumulative toxicities.

(iv) allow improving patient's quality of life during treatments, observance, efficacy of chemotherapy, and patient's outcomes

Furthermore, the experimental section also demonstrated that administration of the claimed amount of C-phycocyanin is also appropriate for the treatment and/or prevention of peripheral neuropathy caused by diabetes (DPN) and infections (such as viral infections -herpes virus and coronavirus infections) in a short period of time after the start of the C-phycocyanin treatment. Again, it is advantageous because allows improving patient's quality of life during treatments, observance, efficacy of diabetes or anti-infections therapy, and patient's outcomes.

Thus, the first aspect the present invention is directed to a product which is C-phycocyanin or an extract comprising C-phycocyanin for use in the treatment and/or prevention of a peripheral neuropathy, wherein the treatment and/or prevention comprises administering C-phycocyanin in an amount from 0.1 to 4.5 mg per Kilogram of body weight per day.

And the second aspect the present invention is directed to a composition comprising the product of the first aspect of the invention and one or more pharmaceutically acceptable excipients or carriers, for use in the treatment and/or prevention of a peripheral neuropathy; wherein the treatment and/or prevention comprises administering C-phycocyanin in an amount from 0.1 to 4.5 mg per Kilogram of body weight per day.

## DETAILED DESCRIPTION OF THE INVENTION

All terms as used herein in this application, unless otherwise stated, shall be understood in their ordinary meaning as known in the art. Other more specific definitions terms as used in the present application are as set forth

below and are intended to apply uniformly throughout the specification and claims unless an otherwise expressly set out definition provides a broader definition.

For the purposes of the present invention, any ranges given include both the lower and the upper endpoints of the range. Ranges given, such as temperatures, times, weights, and the like, should be considered approximate, unless specifically stated. The term "about" or "around" as used herein refers to a range of values $\pm$ 10% of a specified value. For example, the expression "about 0.5" or "around 0.5" includes $\pm$ 10% of 0.5, i.e. from 0.45 to 0.55.

The term "percentage (%) by weight" refers to the percentage of each ingredient in relation to the total weight of the final mixture or composition.

Phycocyanin is a pigment protein complex found in cyanobacteria, Rhodophyceae, and Cryptophyceae and is called C-Phycocyanin (also abbreviated as C-PC) and R-Phycocyanin according to the species. R-Phycocyanin is a minor pigment in red microalgae. The structure is an $(\alpha\beta)_3$ trimer. Both $\alpha$ and $\beta$ subunits possess the phycocyanobilin (PCB) chromophore but the $\beta$ subunit possesses a second chromophore: phycoerythrobilin (PEB).

C-Phycocyanin (C-PC) is a pigment protein complex with a characteristic light blue color, absorbing orange and red light near 620 nm. C-Phycocyanin is the most abundant phycocyanin found in Cyanobacteria, previously called blue green algae. It is a water-soluble heterodimer composed of two polypeptide chains subunits, one $\alpha$ subunit and one $\beta$ subunit with a hexameric conformation $(\alpha\beta)_6$ at pH 5-6 and a trimeric conformation $(\alpha\beta)_3$ at pH 7 (Bryant D.A. et al, Journal of General Microbiology (1982), 128,835-844). These subunits share similar primary amino acid sequences and mainly alpha helix secondary structures. Each subunit chain is covalently attached, respectively, to 2 and 1 molecules of phycocyanobilin (PCB).

The phycocyanobilin is the blue chromophore present in C-PC formed by an open chain including tetrapyrrole rings. The chemical structure of phycocyanobilin is shown below:

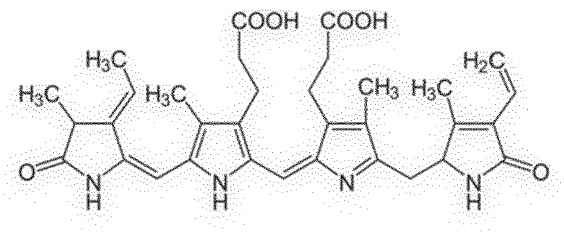

Dimers have molecular weights ranging between 70 and 110 KD. C-PC has a visible absorption maximum between 615 and 620 nm, and a maximum fluorescence emission at 650 nm. These light absorptionemission properties confer it its typical deep blue color and reddish fluorescence. Both are however only found in non-denatured C-PC, with integer apoprotein and its chromophores, phycocyanobilin molecules intact. These heterodimers spontaneously associate in groups of 3 to 6 units as oligomers with circular shapes. Self-assembly into organized, light harvesting antenna is light triggered in algal cells. In these structures, C-PC accepts photons form outer phycoerythrin by fluorescent energy transfer. Through the same process, intermediate phycocyanin passes them down to inner allophycocyanin. From this pigment, photons finally enter the chlorophyll photosynthetic centre, where water molecules are broken and photosynthesis is started.

C-PC fluorescence determines its energy transfer and also its purity. On one hand, the energy transfer value of C-PC has been correlated with its antioxidant activity as ROS scavenger agent. They are dependent on both the association of phycocyanobilin molecules to the intact polypeptide chains and on the light conditions. Indeed, in the dark or under blue light C-PC traps ROS, while under natural white light it generates them. This bi-functional activity seems to be related to conformational changes of the alpha and beta chains. On the other hand, the purity of C-Phycocyanin is generally evaluated using the absorbance ratios $A_{620}/A_{280}$ and $A_{620}/A_{650}$. $A_{620}$ is the maximum absorbance of C-phycocyanin, $A_{650}$ is the maximum absorbance of allophycocyanin and $A_{280}$ is the absorbance of total proteins. A $A_{620}/A_{280}$ ratio of 0.7 is usually considered to define food grade C-phycocyanin, 3.9 as reactive grade and greater than 4.0 as analytical grade (M. Rito-Palomares, L. Nunez, D. Amador, J. Chem. Technol. Biotechnol. 76 (2001) 1273). Figueira et al. Brazilian Journal of Chemical Engineering V. 35, No. 3, pp. 1117-1128 report that a C-phycocyanin with a purity greater than 4 should preferably be used in biomedical applications and as a therapeutic agent. For the purpose of the invention, the purity and concentration of C-PC obtained by extraction, optionally followed by purification steps, can be determined, respectively, by dividing the $A_{620}$ to $A_{280}$ and by using the Equation 1, based on the equation introduced by Bennet in 1973 (cf. J. Cell Biol., 1973, Vol 58, pp. 419-435):

$$\text{Equation 1:} \quad \text{Concentration of C-Phycocyanin} = (A_{620} - 0.474 * A_{652})/5.34$$

C-phycocyanin content is calculated by multiplying the volume and concentration of the solution, together. As it is mentioned above, the first aspect of the invention refers to a product which is C-phycocyanin, or an extract comprising C-phycocyanin, for use in the treatment and/or prevention of a peripheral neuropathy, wherein the treatment and/or prevention comprises administering C-phycocyanin in an amount from 0.1 to 4.5 mg per Kilogram of body weight per day. This aspect may be formulated as the use of the product which is C-phycocyanin or an extract comprising C-phycocyanin as defined herein above and below for the manufacture of a medicament for the treatment and/or prevention of peripheral neuropathy, wherein the treatment and/or prevention comprises administering C-phycocyanin in an amount from 0.1 to 4.5 mg per Kilogram of body weight per day. This aspect may be also formulated as a method for the treatment and/or prevention of a subject in need thereof suffering or susceptible of suffering a peripheral neuropathy, wherein the treatment and/or prevention comprises the administration of a therapeutically effective amount of the product as defined in the first aspect of the invention.

Further, the second aspect of the invention refers to a composition comprising the product as defined in the first aspect of the invention (which is C-phycocyanin or an extract comprising C-phycocyanin) and one or more pharmaceutically acceptable excipients or carriers for use in the treatment and/or prevention of peripheral neuropathy, wherein the treatment and/or prevention comprises administering C-phycocyanin in an amount from 0.1 to 4.5 mg per Kilogram of body weight per day. This aspect may be formulated as the use of the composition comprising the product as defined in the first aspect of the invention (which is C-phycocyanin or an extract comprising C-phycocyanin as defined herein above and below) for the manufacture of a medicament for the treatment and/or prevention of peripheral neuropathy, wherein the treatment and/or prevention comprises administering C-phycocyanin in an amount from 0.1 to 4.5 mg per Kilogram of body weight per day. This aspect may be also formulated as a method for the treatment and/or prevention of a subject in need thereof suffering or susceptible of suffering a peripheral neuropathy, wherein the treatment and/or prevention comprises the administration of a therapeutically effective amount of the product as defined in the first aspect of the invention together with one or more pharmaceutically acceptable excipients or carriers.

In the context of the present invention the term "treatment of peripheral neuropathy" is used to designate the administration of the appropriate amount of C-phycocyanin disclosed herein above and below with the aim of avoiding worsening, reducing, or eliminating the peripheral neuropathy symptoms disclosed above to a subject who is suffering from those peripheral neuropathy symptoms at the time of the administration of the C-phycocyanin.

In the context of the present invention the term "prevention of peripheral neuropathy" is used to designate the administration of the appropriate amount of C-phycocyanin disclosed herein above and below with the aim of avoiding or postponing the appearance of any peripheral neuropathy symptoms disclosed above to a subject who is not suffering from any peripheral neuropathy symptoms at the time of the administration of the C-phycocyanin.

The expression "mg per Kilogram of body weight per day" (or mg/kg/day) refers to the daily dose of C-phytocyanin (expressed in weight; mg) appropriate for being administered to a subject (patient) suffering or susceptible of suffering of peripheral neuropathy according to its body weight expressed in kilograms.

In an embodiment, the product for use of the first aspect of the invention or the composition for use of the second aspect of the invention, is one wherein the treatment and/or prevention comprises administering C-phycocyanin in an amount from 0.1 to 4 mg per Kilogram of body weight per day. In an embodiment, the product for use of the first aspect of the invention or the composition for use of the second aspect of the invention, is one wherein the treatment and/or prevention comprises administering C-phycocyanin in an amount from 0.5 to 4 mg per Kilogram of body weight per day. In an embodiment, the product for use of the first aspect of the invention or the composition for use of the second aspect of the invention, is one wherein the treatment and/or prevention comprises administering C-phycocyanin in an amount from 1 to 4 mg per Kilogram of body weight per day.

In an embodiment, the product for use of the first aspect of the invention or the composition for use of the second aspect of the invention, is one wherein the treatment and/or prevention comprises administering C-phycocyanin in an amount from 0.5 to 4.5 mg per Kilogram of body weight per day; particularly, from 0.5 to 4.5 mg per Kilogram of body weight per day. In an embodiment, the product for use of the first aspect of the invention or the composition for use of the second aspect of the invention, is one wherein the treatment and/or prevention comprises administering C-phycocyanin in an amount from 1 to 4.5 mg per Kilogram of body weight per day.

In a particular embodiment of the first and the second aspect of the invention is one wherein the treatment and/or prevention comprises administering C-phycocyanin in an amount from 0.1 to 4 mg of C-phycocyanin per kilogram of weight of the patient; particularly one or more days.

In an embodiment, the product for use of the first aspect of the invention or the composition for use of the second aspect of the invention, is one wherein the product is C-phycocyanin.

In an embodiment, the product for use of the first aspect of the invention or the composition for use of the second aspect of the invention, wherein the product is an extract comprising C-phycocyanin. It means that the treatment and/or prevention comprises the administration of an appropriate amount of an extract comprising C-phycocyanin corresponding to from 0.1 to 4.5mg of C-phycocyanin per kilogram of body weight of the patient per day. The term "extract" refers to the conventional sense to concentrated preparations of plants, trees, bacteria and/or algae by removing the

active constituents from the plant, tree, bacteria and/or algae with suitable means. Such actives constituents can be obtained from various parts of plants, trees, bacteria and/or algae like for example leaves, root, seed, rhizome, stem, germinated, bacteria or algae cells among others. Suitable means for removal of the active ingredients include, for example, use of organic solvents, microwave, or supercritical fluids extraction. Active ingredients are sometimes directly administered or may be incorporated in a compositions or dietary supplement in a variety of forms, including a pure or semi-pure component, a solid or liquid extract, a dry or wet form. In an embodiment, the extract is a hydrosoluble extract. Plants, tree, bacteria and/or algae extracts contain not only one but multiple constituents, many of them active. Often, the beneficial effect is derived from the combination of many of these active compounds, even though in some cases there is one particular compound that is mainly responsible for most of the activity.

In an embodiment, the product for use of the first aspect of the invention or the composition for use of the second aspect of the invention, is one wherein said product is an extract comprising C-phycocyanin that further comprises at least another phycobiliprotein, particularly selected from the group consisting of allophycocyanin, phycoerythrin, and phycoerythrocyanin. In an embodiment, the product for use of the first aspect of the invention or the composition for use of the second aspect of the invention, is one wherein said product is an extract comprising C-phycocyanin obtained from cyanobacteria.

In an embodiment, the product for use of the first aspect of the invention or the composition for use of the second aspect of the invention, is one wherein said product is an extract comprising C-phycocyanin that further comprises at least another phycobiliprotein, particularly selected from the group consisting of allophycocyanin, phycoerythrin, and phycoerythrocyanin and the extract is obtained from cyanobacteria.

The terms "cyanobacteria", "microalgae" and "Cyanophyta" have the same meaning and are used interchangeably. They refer to a phylum of Gram-negative bacteria that obtain energy via photosynthesis. The name *cyanobacteria* come from their color giving them their other name, "blue-green algae", though modern botanists restrict the term algae to eukaryotes and do not apply it to cyanobacteria, which are prokaryotes. The term "cyanobacteria" encompasses bacteria selected from the group consisting of classes of Chroobacteria, Hormogoneae, and Gloeobacteria; orders of Chroococcales, Gloeobacterales, Nostocales, Oscillatoriales, Pleurocapsales, and Stigonematales; families of Prochloraceae and Prochlorotrichaceae; and genera of *Halospirulina, Planktothricoides, Prochlorococcus, Prochloron,* and *Prochlorothrix.*

In an embodiment, the product for use of the first aspect of the invention or the composition for use of the second aspect of the invention, is one wherein said product is an extract comprising C-phycocyanin obtained from a cyanobacteria, and wherein the cyanobacteria is a *Spirulina algae;* particularly obtained from a cyanobacteria, and wherein the cyanobacteria is *Spirulina Platensis.*

In an embodiment, the product for use of the first aspect of the invention or the composition for use of the second aspect of the invention, is one wherein said product is an extract of C-phycocyanin that further comprises at least another phycobiliprotein and obtained from cyanobacteria, preferably obtained from the cyanobacteria *Spirulina algae; and particularly from Spirulina Platensis.*

For the purpose of the invention, the terms *"Spirulina Platensis" and "Arthrospira platensis" have the same meaning and they are used interchangeably.* They refer to an extract of the cyanobacteria *Spiruline Platensis* that is mainly composed of proteins (64 to 74% by weight of dry matter), 6 to 13% by weight of lipids, of which about half are fatty acids and about 12 to 20% by weight of carbohydrates (A. Vonshak, "Spirulina platensis (Arthrospira)", Taylor & Francis 1997, Chapter 10, pages 175 to 204 ). This extract is a dry extract; particularly a freeze dried extract (lyophilised extract). The Spirulina extract disclosed in the present invention can be commercially available (Spirulysat® supplied by AlgoSource) or may be prepared. The process for its preparation is well known in the state of the art. For the purpose of the invention any of the processes disclosed in the state of the art are appropriate for the preparation of the Spirulina extract of the present invention.

In an embodiment the extract comprising C-phycocyanin is obtained from cyanobacteria of the species *Spirulina Platensis* through a process comprising the following steps:

a) disruption of the cell wall by a method selected from cycles of freezing and thawing, homogenization with mortar and pestle, osmotic shock, treatment with organic or inorganic acids, sonication, high pressure treatment and enzymatic treatment, preferably with a O-glycosylhydrolase.
b) extraction with a solvent, preferably a buffered aqueous solution with a pH comprised between 5 and 7.5
c) centrifugation of the mixture of step b) and separation of the supernatant aqueous solution comprising C-phycocyanin
d) optionally purification of the supernatant aqueous solution obtained in step c)
e) optionally lyophilisation of the purified aqueous solution of step d).

In an embodiment, the product for use of the first aspect of the invention or the composition for use of the second aspect of the invention, is one wherein said product is an extract of C-phycocyanin; particularly obtained from

*Spirulina algae;* comprising an amount equal to or more than 40% by weight of C-phycocyanin. In an embodiment, the product for use of the first aspect of the invention or the composition for use of the second aspect of the invention, is one wherein said product is an extract of C-phycocyanin; particularly obtained from *Spirulina algae;* comprising an amount equal to or more than 60% by weight of C-phycocyanin. In an embodiment, the product for use of the first aspect of the invention or the composition for use of the second aspect of the invention, is one wherein said product is an extract of C-phycocyanin; particularly obtained from *Spirulina algae;* comprising an amount equal to or more than 80% by weight of C-phycocyanin. In an embodiment, the product for use of the first aspect of the invention or the composition for use of the second aspect of the invention, is one wherein said product is an extract of C-phycocyanin; particularly obtained from *Spirulina algae;* comprising an amount equal to or more than 85 % weight of C-phycocyanin. In an embodiment, the product for use of the first aspect of the invention or the composition for use of the second aspect of the invention, is one wherein said product is an extract of C-phycocyanin; particularly obtained from *Spirulina algae;* comprising an amount equal to or more than 90 % weight of C-phycocyanin.

As it is mentioned above, the second aspect the present invention is directed to a composition comprising the product of the first aspect of the invention and one or more pharmaceutically acceptable excipients or carriers, for use in the treatment and/or prevention of a peripheral neuropathy; wherein the treatment and/or prevention comprises administering C-phycocyanin in an amount from 0.1 to 4.5 mg per Kilogram of body weight per day. The term "pharmaceutically acceptable excipients or carriers" refers to that excipients or carriers suitable for use in the pharmaceutical technology for preparing compositions with medical use. The appropriate excipients and/or carriers, their amounts as well as the experimental process conditions for their preparation, can readily be determined by those skilled in the art according to the field and the type of formulation being prepared.

In another particular embodiment, the product of the first aspect of the invention or the composition of the second aspect of the invention are administered orally to the patient to be treated and/or prevented.

In an embodiment, the composition for use of the second aspect of the invention is an oral composition. In an embodiment, the composition for use of the second aspect of the invention is an oral composition selected from liquid oral composition and solid oral composition.

In an embodiment, the composition for use of the second aspect of the invention is an oral solid composition. The oral solid compositions of the invention can be formulated in any form that includes any single unit dosage form and any multiple unit dosage forms. The term "single unit" encompasses one entity such as a single tablet and a single capsule. The term "single unit dosage form" defines a dosage form which consists only of one unit which contains the effective amount of C-phycocyanin. The term "multiple unit dosage form" defines a dosage form which consists of more than one unit which contains the effective amount of C-phycocyanin. Usually the multiple unit dosage forms are based on subunits such as granules, pellets or minitablets. They are usually delivered in hard gelatine capsules or transformed into tablets. In an embodiment, the unit dosage form which comprises the composition of the present invention is a single unit dosage form. In an embodiment, the unit dosage form which comprises the composition of the present invention is a single unit dosage form selected from capsules and reconstituted powder. When the composition is a reconstituted powder suitable for being diluted before use, the powder can be a lyophilized powder. Any method known in the state of the art for the preparation of lyophilized powder for reconstitution are appropriate for the present invention.

In an embodiment, the composition for use of the second aspect of the invention is a liquid oral composition. For the purpose of the invention, the term "liquid" refers to an oral composition intended for oral use wherein the C-phycocyanin and the excipients are partially or totally dissolved in a solvent system; preferably totally dissolved in a solvent system. In an embodiment, the composition for use of the second aspect of the invention is a solution oral liquid composition ready for its use. The liquid solution can be packaged in unitary dose or multidose containers, for example, ampoules, vials, prefilled syringes, cartridges, or bags. The final solution may be dosed and administrated by a syringe, dropper, or any suitable quantitative device.

For the purpose of the invention, the solid oral composition in form of capsules is especially advantageous wherein the treatment and/or prevention of peripheral neuropathy comprises administering C-phycocyanin in an amount from 0.1 to about 0.5mg/kg/day. Furthermore, capsules are easy to prepare, store, handle and administer than the liquid obtained from the powder for reconstitution. Nevertheless, the solid oral composition in form of powder for reconstitution are especially advantageous wherein the treatment and/or prevention of peripheral neuropathy comprises administering C-phycocyanin in an amount from higher than 0.5 to 4.5mg/kg/day. Furthermore, powder for reconstitution is more recommended for patients suffering swelling handicaps or gastrointestinal disease or conditions.

In an embodiment, the composition for use of the second aspect of the invention is an injectable composition. In an embodiment, the composition for use of the second aspect of the invention is an injectable composition selected from the group consisting of intramuscular, subcutaneous, intraperitoneal, or intravenous application. In an embodiment, the composition for use of the second aspect of the invention is in form of parenteral compositions suitable for their injection, infusion, or implantation into the body. The injectable composition defined above should be sterile, and pyrogen-free, and they can be in form of liquid such as solutions, emulsions, or suspensions, or in solid form packaged in either single-dose or multidose containers suitably diluted before use. Injectable compositions can comprise appropriate excipients or carriers for parenteral administration including, but not limited to, solvents, suspending agents, buffering agents, substances to make the preparation isotonic with blood, stabilizers, or antimicrobial preservatives. The addition of excipients should be kept to a minimum. When excipients are used, they should not adversely affect the stability, bioavailability, safety, or efficacy of the polymers and/or the active agents, or cause toxicity or undue local irritation. There should not be any incompatibility between any of the components of the dosage form. The injectable compositions are especially advantageous wherein the treatment and/or prevention comprises a more precise location of the product or composition of the present invention in the target site in a high concentration.

In an embodiment, the composition for use of the second aspect of the invention is a solution of 22.4 mg of the C-phycocyanin extract in 1000 ml of deionized water has UV-visible spectrum showing a ratio $A_{616}/A_{650}$ higher than 3, preferably higher than 3.5 and most preferably higher than 3.61, wherein $A_{616}$ is the absorbance at 616 nm and $A_{650}$, wherein $A_{650}$ is the absorbance at 650 nm. In an embodiment, the composition for use of the second aspect of the invention is one wherein the composition is a liquid composition in form of a solution; particularly, the composition is a liquid composition in form of a solution comprising 22,4 mg of the extract comprising more than 90% by weight of C-phycocyanin in 1000 ml of deionized water showing in the UV-visible spectrum a ratio $A_{616}/A_{650}$ higher than 3, preferably higher than 3.5; and preferably higher than 3.61, wherein $A_{616}$ is the absorbance at 616 nm and $A_{650}$, wherein $A_{650}$ is the absorbance at 650 nm.

In an embodiment, the product for use of the first aspect of the invention or the composition for use of the second aspect of the invention is one wherein the peripheral neuropathy is selected from the group consisting of a chemotherapy induced peripheral neuropathy, radiotherapy induced peripheral neuropathy, infection induced peripheral neuropathy, diabetes induced peripheral neuropathy, atherosclerosis induced peripheral neuropathy, chronic alcohol consumption induced peripheral neuropathy, lung injury induced peripheral neuropathy, kidney injury induced peripheral neuropathy, hepatic injury induced peripheral neuropathy (particularly, non-alcoholic hepatic injury peripheral neuropathy) and a combination thereof. In an embodiment, the product for use of the first aspect of the invention or the composition for use of the second aspect of the invention is one wherein the peripheral neuropathy is selected from the group consisting of a chemotherapy induced peripheral neuropathy, radiotherapy induced peripheral neuropathy, diabetes induced peripheral neuropathy, and a combination thereof. In an embodiment, the product for use of the first aspect of the invention or the composition for use of the second aspect of the invention is one wherein the peripheral neuropathy is selected from the group consisting of a chemotherapy induced peripheral neuropathy, radiotherapy induced peripheral neuropathy, and a combination thereof.

In an embodiment, the product for use of the first aspect of the invention or the composition for use of the second aspect of the invention is one wherein the peripheral neuropathy is a chemotherapy induced peripheral neuropathy. In an embodiment, the product for use of the first aspect of the invention or the composition for use of the second aspect of the invention is one wherein the peripheral neuropathy is a chemotherapy induced peripheral neuropathy, and the chemotherapy comprises administering one or more anti-cancer agents selected form the group consisting of taxane-based anti-cancer agent; a platinum-based anti-cancer agent; a vinca-alkaloid anti-cancer agent; bortezomib; thalidomide and analogs thereof; alkylating anti-cancer agents; and a combination thereof.

In an embodiment the product for use of the first aspect of the invention or the composition for use of the second aspect of the invention is one wherein the peripheral neuropathy is a chemotherapy induced peripheral neuropathy, and the chemotherapy comprises administering one or more anti-cancer agents selected form the group consisting of a taxane-based anti-cancer agent, a platinum-based anti-cancer agent, a vinca-alkaloid anti-cancer agent or combinations thereof. In an embodiment, the product for use of the first aspect of the invention or the composition for use of the second aspect of the invention is one wherein the peripheral neuropathy is a chemotherapy induced peripheral neuropathy, and the chemotherapy comprises administering one or more anti-cancer agents selected form the group consisting of paclitaxel, docetaxel, tesetaxel, cabazitalex, cisplatin, carboplatin, oxaliplatin, vincristine and vinblastine; bortezomib, thalidomide, lenalilomide, and combination thereof. In an embodiment the product for use of the first aspect of the invention or the composition for use of the second aspect of the invention is one wherein the peripheral neuropathy is a chemotherapy induced peripheral neuropathy, and the chemotherapy comprises administering one or more taxane-based anti-cancer agents selected from the group consisting of paclitaxel, docetaxel and cabazitalex. In an embodiment the product for use of the first aspect of the invention or the composition for use of the second aspect of the invention is one wherein the peripheral neuropathy is a chemotherapy induced peripheral neuropathy, and the chemotherapy comprises administering one or more platinum based anti-cancer agents selected from the group consisting of cisplatin, carboplatin and oxaliplatin. In an embodiment the product for use of the first aspect of the invention or the composition for use of the second aspect of the invention is one wherein the peripheral neuropathy is a chemotherapy induced peripheral neuropathy, and the chemotherapy comprises administering one or more vinca alkaloid anti-cancer agents selected from the group consisting of vincristine and vinblastine.

In the context of the present invention the term "treatment of chemotherapy-induced peripheral neuropathy with C-phycocyanin" is used to designate the administration of the appropriate amount of C-phycocyanin disclosed herein above and below with the aim of avoiding worsening, reducing, or eliminating the symptoms of chemotherapy-induced peripheral neuropathy in a subject who has received or is receiving a chemotherapeutic treatment and who shows one or more of said peripheral neuropathy symptoms at the time C-phycocyanin starts to be administered to said patient. In the context of the present invention the term "prevention of chemotherapy-induced peripheral neuropathy with C-phycocyanin" is used to designate the administration of the appropriate amount of C-phycocyanin disclosed herein above and below with the aim of avoiding or postponing the appearance of any chemotherapy-induced peripheral neuropathy symptoms disclosed above to a subject who has received, is receiving or is about to receive a chemotherapeutic treatment and does not shows symptoms of chemotherapy-induced peripheral neuropathy at the time C-phycocyanin starts to be administered to said patient.

The above mentioned prevention and/or treatment involve the administration of an effective dose of C-phycocyanin to a subject in need thereof, which is from 0.1 to 4.5 mg per Kilogram of body weight per day according to the present invention. It is possible to administer the above amount of C-phycocyanin every day during the chemotherapy treatment but is also possible to administer said amount only some days during the chemotherapy treatment. For example, when the treatment with C-phycocyanin is administered during a period overlapping completely or partially with the period when the patient is receiving chemotherapeutic treatment it may be advantageous to administer said C-phycocyanin on days in which the patient does not receive any dose of the chemotherapeutic treatment. Thus, in an embodiment the product for use of the first aspect of the invention or the composition for use of the second aspect of the invention is one wherein the peripheral neuropathy is a chemotherapy induced peripheral neuropathy and the treatment and/or prevention comprises administering C-phycocyanin during a period overlapping completely or partially with the period during which the patient is receiving chemotherapeutic treatment. In an embodiment, the product for use of the first aspect of the invention or the composition for use of the second aspect of the invention is one wherein the peripheral neuropathy is a chemotherapy induced peripheral neuropathy and the treatment and/or prevention comprises administering C-phycocyanin on days in which the patient does not receive any dose of the chemotherapeutic treatment.

In an embodiment, the product for use of the first aspect of the invention or the composition for use of the second aspect of the invention is one wherein the peripheral neuropathy is a chemotherapy induced peripheral neuropathy and the treatment and/or prevention comprises administering C-phycocyanin before the start of administration of the chemotherapy. In an embodiment the product for use of the first aspect of the invention or the composition for use of the second aspect of the invention is one wherein the peripheral neuropathy is a chemotherapy induced peripheral neuropathy and the treatment and/or prevention comprises administering the C-phycocyanin after the start of administration of the chemotherapy. In an embodiment the product for use of the first aspect of the invention or the composition for use of the second aspect of the invention is one wherein the peripheral neuropathy is a chemotherapy induced peripheral neuropathy and the treatment and/or prevention comprises administering the C-phycocyanin after the end of chemotherapy.

In an embodiment the product for use of the first aspect of the invention or the composition for use of the second aspect of the invention is one wherein the peripheral neuropathy is a chemotherapy induced peripheral neuropathy and the treatment and/or prevention comprises administering the C-phycocyanin after the start of administration of the chemotherapy, wherein: the appropriate amount of C-phytocyanin is administered after the end of the administration of the chemotherapy, and at least on or from the day after the chemotherapy corresponding to the double of the half-life of the anti-cancer agent. It means that, for example, the administration of the platinum-based anti-cancer agent, cisplatin, which has a plasma half-life of 30min, is recommended at least 3 days (more than double of the plasma half-life) after the end of the chemotherapy.

In an embodiment, the product for use of the first aspect of the invention or the composition for use of the second aspect of the invention is one wherein the peripheral neuropathy is a chemotherapy induced peripheral neuropathy and the treatment and/or prevention comprises: firstly, administering C-phycocyanin before the chemotherapeutic treatment; and secondly administering C-phycocyanin after the end of the chemotherapeutic treatment as defined herein above and below.

In an embodiment, the product for use of the first aspect of the invention or the composition for use of the second aspect of the invention is one wherein the peripheral neuropathy is a diabetes induced peripheral neuropathy. In an embodiment, the product for use of the first aspect of the invention or the composition for use of the second aspect of the invention is one wherein the peripheral neuropathy is a diabetes induced peripheral neuropathy and the treatment and/or prevention comprises administering C-phycocyanin simultaneously, sequentially or separately from the antidiabetic treatment. Regardless the administration regimen, the effectiveness of the treatment of the present invention for DPN is maintained.

In an embodiment, the product for use of the first aspect of the invention or the composition for use of the second aspect of the invention is one wherein the peripheral neuropathy is an infection induced peripheral neuropathy. In an embodiment, the product for use of the first aspect of the invention or the composition for use of the second aspect of the invention is one wherein the peripheral neuropathy is an infection induced peripheral neuropathy selected from the group consisting of viral induced peripheral neuropathy, bacterial induced peripheral neuropathy and parasitic induced peripheral neuropathy. In an embodiment, the product for use of the first aspect of the invention or the composition for

use of the second aspect of the invention is one wherein the peripheral neuropathy is a viral infection induced peripheral neuropathy.

In an embodiment, the product for use of the first aspect of the invention or the composition for use of the second aspect of the invention is one wherein the peripheral neuropathy is a viral infection induced peripheral neuropathy selected from the group consisting of Human immune deficiency virus (HIV), Human T-cell lymphotropic virus (HTLV), Herpes simplex (HSV 1 and 2), Varicella zoster Virus (VZV), Cytomegalovirus (CMV), Epstein-Barr virus (EBV), West Nile virus (WNV), Hepatitis C virus (HCV), Rabies virus (RV), severe acute respiratory syndrome coronavirus 1 (SARS-CoV-1), severe acute respiratory syndrome coronavirus 2 (SARS-CoV-2), Middle East respiratory syndrome coronavirus (MERS-CoV); and Coronavirus disease 2019 (COVID-19).

In an embodiment, the product for use of the first aspect of the invention or the composition for use of the second aspect of the invention is one wherein the peripheral neuropathy is a herpes viral infection induced peripheral neuropathy selected from the group consisting of Herpes simplex (HSV 1 and 2) and Varicella zoster Virus (VZV).

In an embodiment, the product for use of the first aspect of the invention or the composition for use of the second aspect of the invention is one wherein the peripheral neuropathy is a herpes viral infection induced peripheral neuropathy selected from the group consisting of severe acute respiratory syndrome coronavirus 1 (SARS-CoV-1), severe acute respiratory syndrome coronavirus 2 (SARS-CoV-2), Middle East respiratory syndrome coronavirus (MERS-CoV); and Coronavirus disease 2019 (COVID-19); particularly, Coronavirus disease 2019 (COVID-19).

In an embodiment, the product for use of the first aspect of the invention or the composition for use of the second aspect of the invention is one wherein the peripheral neuropathy is an infection induced peripheral neuropathy as defined above and the treatment and/or prevention comprises administering C-phycocyanin simultaneously, sequentially or separately from the antiinfection treatment.

In an embodiment, the product for use of the first aspect of the invention or the composition for use of the second aspect of the invention is one wherein the peripheral neuropathy is an infection induced peripheral neuropathy, wherein the infection induced peripheral neuropathy is a virus induced peripheral neuropathy, particularly Herpes virus peripheral neuropathy or coronavirus peripheral neuropathy, as defined above and the treatment and/or prevention comprises administering C-phycocyanin simultaneously, sequentially or separately from the antiviral infection treatment. Regardless the administration regimen, the effectiveness of the treatment of the present invention is maintained.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

## Examples

### 1. Treatment and/or prevention of peripheral neuropathy with C-phycocyanin

#### 1.1. General considerations

The degree of peripheral neuropathy (PN) suffered by the patient was assessed by the specialist visiting the patients and assigned to one of the following levels:

- PN0: None: No peripheral neuropathy symptoms
- PN1: Minimal: presence of paraesthesia (tingle, loss of tendon reflexes)
- PN2: Moderate: Moderate to severe paraesthesia (loss of sensitivity, pins and needles, temperature sensitivity with or without pain)
- PN3: Severe: Incapacitating paraesthesia, interfering with function
- PN4: Loss of function: Severe sensory loss, paralysis, marked weakness

#### 1.2. Treatment and/or prevention of peripheral neuropathy by administration of enriched (concentrated) extract of *Arthrospira platensis*

In the following examples 1-34 C-phycocyanin was administered to patients suffering from or susceptible of suffering a peripheral neuropathy in the form of an aqueous solution comprising the desired daily dose of C-phycocyanin according to the present invention.

The solution was prepared by dissolving of sufficient quantity of a product consisting of a mixture comprising 500 mg of a lyophilised extract from *Arthrospira platensis* containing with a titrated content of C-phycocyanin of 90.8%

calculated using the formula of Equation 1 reported above and 500 mg of excipients (maltodextrin and magnesium stearate) in mineral water just before oral administration to obtain an aqueous solution of approximately 3, 6 or 8 mg of C-phycocyanin/ml of solution. As it is disclosed herein below, an effective amount from 0.25 mg to 4.5 mg of C-phytocyanin/kg/day was administered.

### 1.2.1. Treatment and/or prevention of chemotherapy-induced peripheral neuropathy

The utility of C-phycocyanin in the treatment and/or prevention of chemotherapy-induced peripheral neuropathy is shown in the following examples.

**Experimental data compiled from December 2017 to July 2020**

The data shown in examples 1-19 was compiled from patients treated by oncologists at different hospitals during the period ranging from December 2017 to July 2020. All patients had been diagnosed cancer by an oncologist and received chemotherapy either before or during the treatment with C-phycocyanin. None of the patients suffered from diabetes.

The degree of peripheral neuropathy (PN) suffered by the patient was assessed by an oncologist visiting the patients and assigned from PN0 to PN4 levels as mentioned above.

- Chemotherapeutical treatment

The dose of the chemotherapeutic active ingredient is expressed as mg of drug/$m^2$ body surface; the days of administration are expressed as Dx (for example D1 for the first day, D2 for the second day of treatment and subsequently); and the chemotherapy cycles are expressed as Cx (for example C1 for the first cycle, C2 for the second cycle and subsequently).

The doses and posology of the chemotherapeutic drug were as follows:

FOLFOX4: Folinic acid 200 D1-2 + 5-FluoroUracyl 400 D1-2 + Oxaliplatin 85 D1

TAXOL: 80 mg/m2/week

CG: Cisplatin 70 mg/m2 D1 + Gemcitabine 1,000 mg/m2 D1, D7

TAXOL/Carboplatin: Taxol 175 mg/m2 + Carboplatin AUC (Calvert formula)

Carboplatin and Cisplatin administered every 21 days

**Patient 1 (treatment):**

Patient data

Male, 68 years, 1.80 m height, 74 Kg weight, Body Mass Index 23
Prior conditions: Tobacco smoker until 20 years before treatment, Bipolar disease under treatment, diverticulosis

Cancer data

Cancer diagnostic: Urothelial cancer, stage IV
Chemotherapy regime: Cisplatin 70 mg/$m^2$ (Day 1), Gemcitabine 1,000 mg/$m^2$ (Days 1 and 7)
Number of cycles: 6 after surgery (right nephrectomy)
Comments: pulmonary metastasis, largest 11 mm, right nephrectomy (2016)

C-Phycocyanin treatment data

Start of C-phycocyanin treatment: 1 year after end of chemotherapy
Neuropathy grade before start of C-phycocyanin treatment: PN3 (in feet)
C-Phycocyanin dosage: 1 mg/kg/day

C-Phycocyanin treatment period: 2 month starting July 2019 + 2 months starting March 2020
Neuropathy grade after C-phycocyanin treatment: PN0
Conclusion: C-Phycocyanin treatment achieved a reduction of peripheral neuropathy from PN3 to PN0

**Patient 2 (prevention):**

Patient data

Male, 74 years, 1.75m height, 80 Kg weight, Body Mass Index 26
Prior conditions: Tobacco smoker, hypertension

Cancer data

Cancer diagnostic: Urothelial cancer, stage III
Chemotherapy regime: Cisplatin 70 mg/m$^2$ (Day 1), Gemcitabine 1,000 mg/m$^2$ (Days 1 and 7)
Number of cycles: 3 before surgery (cystectomy)

C-Phycocyanin treatment data

Start of C-phycocyanin treatment: before start of chemotherapy
Neuropathy grade before start of C-phycocyanin treatment: PN0
C-Phycocyanin dosage: day before start of chemotherapy 3.75 mg/kg/day, from day 4 after start of each chemotherapeutic cycle until 2 days before next chemotherapeutic cycle 1 mg/kg/day
C-Phycocyanin treatment period: 4 month starting October 2019 + 4 months starting January 2020 Neuropathy grade after C-phycocyanin treatment: PN0
Conclusion: C-Phycocyanin treatment prevented the development of peripheral neuropathy

**Patient 3 (treatment):**

Patient data

Male, 56 years, 1.70 m height, 79 Kg weight, Body Mass Index 27
Prior conditions: Tobacco smoker, overweight, diverticulitis, bilateral inguinal hernia

Cancer data

Cancer diagnostic: Mucinous Adeno Carcinoma of the colon, stage IVB
Chemotherapy regime: Folinic acid 200 mg/m$^2$ (days 1-2) + 5-Fluorouracyl 400 mg/m$^2$ (days 1-2) + Oxaliplatin 85 mg/m$^2$ (day 1)
Number of cycles: 4 before surgery
Comments: Surgery in July 2020: no possible debulking, extensive peritoneal carcinomatosis, mucinous adenocarcinoma, Hernia repair

C-Phycocyanin treatment data

Start of C- phycocyanin treatment: After completing 2$^{nd}$ cycle of chemotherapy
Neuropathy grade before start of C-phycocyanin treatment: PN3 (in hands)
C-Phycocyanin dosage: 1.125 mg/kg/day
C-Phycocyanin treatment period: 2 month starting after completing 2$^{nd}$ chemotherapy cycle + 2 months starting July 2020
Neuropathy grade after C-phycocyanin treatment: PN0
Conclusion: C-Phycocyanin treatment achieved a reduction of peripheral neuropathy from PN3 to PN0

**Patient 4 (treatment):**

Patient data

Female, 64 years, 1.66 m height, 69 Kg weight, Body Mass Index 25
Prior conditions: Sleep apnoea, Tobacco smoker until she was 25 years old, cardiopathy (Ejection Fraction 49%)

Cancer data

Cancer diagnostic: Breast cancer, stage Ia
Chemotherapy regime: Paclitaxel (80 $mg/m^2$/week) / Trastuzumab (every three weeks for a year at 8 mg/kg) after surgery
Number of cycles: 12 before surgery, 6 after surgery
Comments: Previous surgery April 2019, Adenocarcinoma, pT2N0M0, HER2 +++, ER 95% RP 80%, Ki67 50%, 4 previous cycles with Epirubicine plus Cyclophosphamide

C-Phycocyanin treatment data

Start of C-phycocyanin treatment: After completing cycle 9 of paclitaxel and cycle 3 of Trastuzumab Neuropathy grade before start of C-phycocyanin treatment: PN3 (in hands)
C-Phycocyanin dosage: 1 mg/kg/day on days 2 and 6 of each cycle
C-Phycocyanin treatment period: 5 months
Neuropathy grade after C-phycocyanin treatment: PN0
Conclusion: C-Phycocyanin treatment achieved a reduction of peripheral neuropathy from PN3 to PN0

**Patient 5 (prevention):**

Patient data

Female, 45 years, 1.69 m height, 58 Kg weight, Body Mass Index 20
Prior conditions: Chronic ferropenic anaemia, menorrhagia, endometriosis

Cancer data

Cancer diagnostic: Breast cancer, stage II
Chemotherapy regime: paclitaxel (80 $mg/m^2$/week) / Trastuzumab (every three weeks for a year at 6 mg/kg) after surgery
Number of cycles: 12 before surgery, 6 after surgery (
Comments: Previous surgery in June 2019, Adenocarcinoma, pT1aN1M0, ER-, PR-, HER2+, Ki67 50%, 4 previous cycles with Epirubicine plus Cyclophosphamide, post RT

C-Phycocyanin treatment data

Start of C-phycocyanin treatment: Just before starting chemotherapy
Neuropathy grade before start of C-phycocyanin treatment: PN0 (not yet treated with chemotherapy)
C-Phycocyanin dosage: 1 mg/kg/day on days 2 to 5 of each chemotherapy cycle and 2.5 mg/kg/day on day 6 of each chemotherapy cycle
C-Phycocyanin treatment period: 9 months
Neuropathy grade after C-phycocyanin treatment: PN0/PN1 (some insensitivity in fingers of the feet)
Conclusion: C-Phycocyanin treatment controlled the appearance of peripheral neuropathy (PN0/PN1) after aggressive chemotherapy.

**Patient 6 (treatment):**

Patient data

Male, 66 years, 1.82 m height, 72 Kg weight, Body Mass Index 22
Prior conditions: Cholecystectomy, Benign prostate hypertrophy, arthrosis

Cancer data

Cancer diagnostic: Colon Adeno Carcinoma, stage IVB
Chemotherapy regime: Folinic acid 200 mg/$m^2$ (days 1-2) + 5-Fluorouracyl 400 mg/$m^2$ (days 1-2) + Oxaliplatin 85 mg/$m^2$ (day 1)
Number of cycles: 8 before surgery and 4 after surgery
Comments: Pulmonary and hepatic Metastasis, KRAS mutated, colostomy May 2018, lobectomy June 2018

C-Phycocyanin treatment data

Start of C-phycocyanin treatment: 1 year after completing chemotherapy
Neuropathy grade before start of C-phycocyanin treatment: PN2 (hands and feet)
C-Phycocyanin dosage: 0.25 mg/kg/day on days 6-12 of each month
C-Phycocyanin treatment period: 9 months
Neuropathy grade after C-phycocyanin treatment: PN1 (only in feet)
Conclusion: C-Phycocyanin treatment achieved a reduction of peripheral neuropathy from PN2 in hands and feet to PN0 in hands and to PN1 in feet

**Patient 7 (prevention):**

Patient data

Female, 60 years, 1.65 m height, 65 Kg weight, Body Mass Index 22
Prior conditions: Hypertension treated with atenolol

Cancer data

Cancer diagnostic: Rectal Adeno Carcinoma, stage IIIA
Chemotherapy regime: Folinic acid 200 mg/$m^2$ (days 1-2) + 5-Fluorouracyl 400 mg/$m^2$ (days 1-2) + Oxaliplatin 85 mg/$m^2$ (day 1)
Number of cycles: 2 after surgery
Comments: Ileostomy bag

C-Phycocyanin treatment data

Start of C-phycocyanin treatment: just before starting chemotherapy
Neuropathy grade before start of C-phycocyanin treatment: PN0
C-Phycocyanin dosage: 1 mg/kg/day on days 6-12 of each month
C-Phycocyanin treatment period: 2 months
Neuropathy grade after C-phycocyanin treatment: PN0
Conclusion: C-Phycocyanin treatment controlled the appearance of peripheral neuropathy (PNO) after aggressive chemotherapy.

**Patient 8 (prevention):**

Patient data

Female, 54 years, 1.55 m height, 44 Kg weight, Body Mass Index 18
Prior conditions: Atypical appendicectomy in 1992

Cancer data

Cancer diagnostic: Mucinous Adeno Carcinoma of the colon, stage IVB
Chemotherapy regime: Folinic acid 200 mg/m$^2$ (days 1-2) + 5-Fluorouracyl 400 mg/m$^2$ (days 1-2) + Oxaliplatin 85 mg/m$^2$ (day 1) + Bevacizumab (bi-weekly, 5 mg/kg)
Number of cycles: 12 after surgery
Comments: Peritoneal carcinomatosis, 2 cytoreduction surgeries in March 2017 and April 2020

C-Phycocyanin treatment data

Start of C-phycocyanin treatment: Just before starting chemotherapy
Neuropathy grade before start of C-phycocyanin treatment: PN0
C-Phycocyanin dosage: 0.5 mg/kg/day on days 6-12 of each month and 1 mg/kg/day on day 13 of each month
Neuropathy grade after C-phycocyanin treatment: PN0
Conclusion: C-Phycocyanin treatment controlled the appearance of peripheral neuropathy (PNO) after aggressive chemotherapy.

**Patient 9 (prevention):**

Patient data

Female, 51 years, 1.63 m height, 50 Kg weight, Body Mass Index 19
Prior conditions: Diagnosis on May 2018, already in stage IV

Cancer data

Cancer diagnostic: Colon Adeno Carcinoma, stage IVB
Chemotherapy regime: Folinic acid 200 mg/m$^2$ (days 1-2) + 5-Fluorouracyl 400 mg/m$^2$ (days 1-2) + Oxaliplatin 85 mg/m$^2$ (day 1)
Number of cycles: 2 separated 14 days
Comments: Pulmonary, hepatic, and bone metastasis, malignant ascitis

C-Phycocyanin treatment data

Start of C-phycocyanin treatment: Just before starting chemotherapy
Neuropathy grade before start of C-phycocyanin treatment: PN0
C-Phycocyanin dosage: 0.5 mg/kg/day on days 6-12 of each month and 1 mg/kg/day on day 13 of each month
C-Phycocyanin treatment period: 1 months
Neuropathy grade after C-phycocyanin treatment: PN0
Conclusion: C-Phycocyanin treatment controlled the appearance of peripheral neuropathy (PNO) after aggressive chemotherapy.

**Patient 10 (prevention):**

Patient data

Female, 61 years, 1.58 m height, 65 Kg weight, Body Mass Index 26
Prior conditions: moderate overweight, hypertension

Cancer data

Cancer diagnostic: Endometrium Carcino-sarcoma, stage IV
Chemotherapy regime paclitaxel 80 mg/$m^2$/week on days 1, 7, 15/28
Number of cycles: 3
Comments: hysterectomy, adnexectomy, complete pelvic lymphadenectomy, February 2018, Peritoneal carcinomatosis (found April 2019)

C-Phycocyanin treatment data

Start of C-phycocyanin treatment: Just before starting chemotherapy
Neuropathy grade before start of C-phycocyanin treatment: PN0
C-Phycocyanin dosage: 0.5 mg/kg/day daily except on days 1, 7 and 15 of each cycle
C-Phycocyanin treatment period: 3 months
Neuropathy grade after C-phycocyanin treatment: PN0/PN1 (some insensitivity in toes)
Conclusion: C-Phycocyanin treatment controlled the appearance of peripheral neuropathy (from PN0 to PN1) after aggressive chemotherapy.

**Patient 11 (treatment):**

Patient data

Female, 77 years, 1.73 m height, 60 Kg weight, Body Mass Index 20
Prior conditions: Human Papilloma virus positive in rectum

Cancer data

Cancer diagnostic Invasive squamous carcinoma of the rectum T2N0M0 (T2: tumour size, N0: no lymphatic glands affected, M0: no distant (metastatic) lesions detected by image (PET/TC scanner)), stage I
Chemotherapy regime: Concomitant radiotherapy with cisplatin (CDDP) 70 mg/$m^2$ + 5 Fluorouracil for 3 days 400 mg/$m^2$ in total
Number of cycles: 1 before surgery
Comments: Chemotherapy stopped at first cycle because of severe side effects (neuropathy, hearing loss, weight loss), tumour treated with radiotherapy

C-Phycocyanin treatment data

Start of C-phycocyanin treatment: 3.5 years after end of chemotherapy
Neuropathy grade before start of C-phycocyanin treatment: PN1 (in feet + hearing loss)
C-Phycocyanin dosage: 0.25 mg/kg/day
C-Phycocyanin treatment period: 10 months
Neuropathy grade after C-phycocyanin treatment: PN0
Conclusion: C-Phycocyanin treatment achieved a reduction of peripheral neuropathy from PN1 to PN0

**Patient 12 (treatment):**

Patient data

Male, 83 years, 1.78 m height, 97 Kg weight, Body Mass Index 31
Prior conditions: overweight, hypertension, dyslipidaemia, depression

Cancer data

Cancer diagnostic: colon adenocarcinoma, stage III

Chemotherapy regime: Folinic acid 200 mg/m$^2$ (days 1-2) + 5-Fluorouracyl 400 mg/m$^2$ (days 1-2) + Oxaliplatin 85 mg/m$^2$ (day 1)
Number of cycles: 12 after surgery but the last 4 of them without oxaliplatin

C-Phycocyanin treatment data

Start of C-phycocyanin treatment: 3.5 years after end of chemotherapy
Neuropathy grade before start of C-phycocyanin treatment: PN1 (in feet)
C-Phycocyanin dosage: initially 0.30 mg/kg/day, then 1 mg/kg/day
C-Phycocyanin treatment period: 12 months
Comments: C-Phycocyanin treatment from December 2015 to June 2019 was done with a lower phycocyanin dose
Neuropathy grade after C-phycocyanin treatment: PN0
Conclusion: C-Phycocyanin treatment achieved a reduction of peripheral neuropathy from PN1 to PN0

**Patient 13 (prevention):**

Patient data

Female, 58 years, 1.65 m height, 67 Kg weight, Body Mass Index 25
Prior conditions: endometriosis, intestinal occlusion, intestinal problems, family deafness, rheumatoid arthritis

Cancer data

Cancer diagnostic: ovary mucinous carcinoma, stage II
Chemotherapy regime: Paclitaxel 175 mg/m$^2$ +Carboplatin AUC (Calvert formula)
Number of cycles: 6 after surgery
Comments: Rheumatoid arthritis improved during treatment

C-Phycocyanin treatment data

Start of C-phycocyanin treatment: when starting chemotherapy
Neuropathy grade before start of C-phycocyanin treatment: PN0
C-Phycocyanin dosage: 0.25 mg/kg/day
C-Phycocyanin treatment period: 10 months
Neuropathy grade after C-phycocyanin treatment: PN0
Conclusion: C-Phycocyanin treatment controlled the appearance of peripheral neuropathy (PNO) after aggressive chemotherapy.

**Patient 14 (prevention):**

Patient data

Female, 59 years, 1.70 m height, 60 Kg weight, Body Mass Index 21
Prior conditions: sinus inflammation, allergies

Cancer data

Cancer diagnostic: invasive ductal carcinoma of the breast (HER2+), stage II
Chemotherapy regime: Carboplatin 900 mg 5 cycles /Docetaxel 75 mg/m$^2$ 5 cycles /Pertuzumab 840 mg in 1$^{st}$ cycle and 420 mg in following cycles each 21 days /Trastuzumab 8 mg/m$^2$ in 1$^{st}$ cycle and 6 mg/m2 in following cycles each 21 days
Number of cycles: 5 after surgery
Comments: previous treatment with Pertuzumab + Trastuzumab neoadjuvant, followed by surgery in January

2019 (tumorectomy + adenectomy) then radiotherapy, and chemotherapy

C-Phycocyanin treatment data

Start of C-phycocyanin treatment: when starting chemotherapy
Neuropathy grade before start of C-phycocyanin treatment: PN0
C-Phycocyanin dosage: 0.75 mg/kg/day
C-Phycocyanin treatment period: 8 months
Neuropathy grade after C-phycocyanin treatment: PN0
Conclusion: C-Phycocyanin treatment controlled the appearance of peripheral neuropathy after aggressive chemotherapy

**Patient 15 (treatment):**

Patient data

Female, 70 years, 1.57 m height, 51 Kg weight, Body Mass Index 21
Prior conditions: thrombosis at 30 years old, treated with acetyl salicylic acid 100 mg

Cancer data

Cancer diagnostic: Infiltrating breast ductal carcinoma, stage IV
Chemotherapy regime: oral Tesetaxel 30 mg /Capecitabine 800 mg during 3 weeks plus letrozole (2.5 mg daily, orally)
Number of cycles: 9 after surgery
Comments: surgery in 2011 (mastectomy, 14 affected nodes), malignant myocarditis (2018), start of clinical assay 4/2019: Tesetaxel 30 mg + Capecitabine 800 mg/3 weeks

C-Phycocyanin treatment data

Start of C-phycocyanin treatment: after end of chemotherapy
Neuropathy grade before start of C-phycocyanin treatment: PN3 (feet, legs, and hands)
C-Phycocyanin dosage: 1.5 mg/kg/day
C-Phycocyanin treatment period: 7 months
Neuropathy grade after C-phycocyanin treatment: PN1 (in fingers and toes)
Conclusion: C-Phycocyanin treatment reduced peripheral neuropathy (from PN3 to PN1) after aggressive chemotherapy

**Patient 16 (treatment):**

Patient data

Female, 58 years, 1.69 m height, 59 Kg weight, Body Mass Index 21
Prior conditions: ovarian cyst, precocious menopause (at 31), hormonal therapy for 23 years, severe family stress for 20 years.

Cancer data

Cancer diagnostic: Ovarian cancer, epithelial clear cells, stage III
Chemotherapy regime: Paclitaxel 175 mg/$m^2$ + Carboplatin AUC (Calvert formula)
Number of cycles: 4 after surgery
Comments: First resection right ovary December 2017, later complete hysterectomy, omentectomy and pelvic lymphadenectomy in January 2018

C-Phycocyanin treatment data

Start of C-phycocyanin treatment: after end of chemotherapy
Neuropathy grade before start of C-phycocyanin treatment: PN3 (in feet, legs, and hands) C-Phycocyanin dosage: 0.25 mg/kg/day
C-Phycocyanin treatment period: 12 months
Neuropathy grade after C-phycocyanin treatment: PN0
Conclusion: C-Phycocyanin treatment reduced peripheral neuropathy (from PN3 to PN0).

**Patient 17 (treatment):**

Patient data

Female, 54 years, 1.57 m height, 48 Kg weight, Body Mass Index 19
Prior conditions: No children, chronic stress, early menarchia.

Cancer data

Cancer diagnostic: invasive ductal carcinoma of the breast, stage III
Chemotherapy regime: Paclitaxel 80 mg/$m^2$/week
Number of cycles: 12 before surgery
Comments: First resection right ovary December 2017, later complete hysterectomy, omentectomy and pelvic lymphadenectomy in January 2018

C-Phycocyanin treatment data

Start of C-phycocyanin treatment: after end of chemotherapy
Neuropathy grade before start of C-phycocyanin treatment: PN3 (in feet and legs)
C-Phycocyanin dosage: 0.5 mg/kg/day
C-Phycocyanin treatment period: 14 months
Neuropathy grade after C-phycocyanin treatment: PN0
Conclusion: C-Phycocyanin treatment reduced peripheral neuropathy (from PN3 to PN0).

**Patient 18 (treatment):**

Patient data

Female, 63 years, 1.69 m height, 75 Kg weight, Body Mass Index 26
Prior conditions: cholelithiasis, hysterectomy at 45 (uterine fibroma), chronic severe family/social stress.

Cancer data

Cancer diagnostic: Infiltrating breast ductal carcinoma, stage III
Chemotherapy regime: Paclitaxel 80 mg/$m^2$/week
Number of cycles: 12 before surgery
Comments: Triple negative, multifocal, luminal B, 4 previous cycles with Epirubicine plus Cyclophosphamide, gall bladder surgery during chemotherapy in July 2019

C-Phycocyanin treatment data

Start of C-phycocyanin treatment: before the start of chemotherapy
Neuropathy grade before start of C-phycocyanin treatment: PN3 (in feet, legs, and hands)
C-Phycocyanin dosage: 0.5 mg/kg/day
C-Phycocyanin treatment period: 8 months

Neuropathy grade after C-phycocyanin treatment: PN1 (in feet and legs)
Conclusion: C-Phycocyanin treatment reduced peripheral neuropathy (from PN3 to PN1).

**Patient 19 (treatment):**

Patient data

Female, 49 years, 1.83 m height, 68 Kg weight, Body Mass Index 20
Prior conditions: breast fibroadenomas, family stress.

Cancer data

Cancer diagnostic: Infiltrating breast ductal carcinoma, stage III
Chemotherapy regime: Paclitaxel (135mg/$m^2$)/Carboplatin(600 mg total)/Bevacizumab(days 1 and 15 every 28 days, 10 mg/kg)/Triptorelin3.75 mg monthly)
Number of cycles: 12 before surgery
Comments: mastectomy and lymphadenectomy in July 2016

C-Phycocyanin treatment data

Start of C-phycocyanin treatment: after end of chemotherapy
Neuropathy grade before start of C-phycocyanin treatment: PN3 (in feet and legs)
C-Phycocyanin dosage: 0.5 mg/kg/day
C-Phycocyanin treatment period: 3 months
Neuropathy grade after C-phycocyanin treatment: PN0
Conclusion: C-Phycocyanin treatment reduced peripheral neuropathy (from PN3 to PN0).

**Experimental data compiled from July 2020 to September 2021**

The data shown in examples 20-30 was compiled from patients treated by oncologists at different hospitals during the period ranging from July 2020 to September 2021. All patients had been diagnosed cancer by an oncologist and received chemotherapy either before or during the treatment with C-phycocyanin. None of the patients suffered from diabetes.

The degree of peripheral neuropathy (PN) suffered by the patient was assessed by an oncologist visiting the patients and assigned from PN0 to PN4 levels as mentioned above. And the chemotherapeutical treatment details are the same mentioned in previous section for patients 1-18.

The patient information (sex, Age, Height, weight and Body Mass Index (BMI; measured as disclosed in Keys A. et al. "Indices of relative weight and obesity". International Journal of Epidemiology, 2014, vol 43(3), pp.655-665) and patient clinical conditions (prior clinical conditions, cancer diagnostic and stage at diagnosis) are summarized in the following Table:

| **Patient Information** | | | | | | **Patient Clinical Condition** | | |
|---|---|---|---|---|---|---|---|---|
| **Patient number** | **Sex** | **Age** | **Height (m)** | **Weight (kg)** | **BMI** | **Prior Conditions** | **Cancer Diagnostic** | **Stage at Diagnosis** |
| **PATIENT 20** | Female | 70 | 1,68 | 82 | 29 | Myomas, colon polyps, overweight | Infiltrating breast ductal carcinoma | II |
| **PATIENT 21** | Female | 50 | 1,80 | 62 | 19 | None | Infiltrating breast ductal carcinoma | IA |

(continued)

| Patient Information | | | | | | Patient Clinical Condition | | |
|---|---|---|---|---|---|---|---|---|
| **Patient number** | **Sex** | **Age** | **Height (m)** | **Weight (kg)** | **BMI** | **Prior Conditions** | **Cancer Diagnostic** | **Stage at Diagnosis** |
| **PATIENT 22** | Female | 62 | 1,60 | 57 | 22 | None | Rectum AC | II |
| **PATIENT 23** | Female | 61 | 1,55 | 71 | 30 | Hepatitis B (since 1995) | Infiltrating Colon AC | I |
| **PATIENT 24** | Female | 64 | 1,50 | 53 | 24 | osteoporosis, renal lithiasis, ovary cysts | Breast AC | II |
| **PATIENT 25** | Male | 61 | 1,81 | 67 | 20 | Polymedication, diabetes ID | Pancreas AC | III |
| **PATIENT 26** | Female | 60 | 1,63 | 51 | 19 | Multinodular goiter, fatty liver | Pancreas AC | I |
| **PATIENT 27** | Female | 45 | 1,56 | 49 | 20 | None | Colon AC | I |
| **PATIENT 28** | Female | 43 | 1,58 | 42 | 17 | Fibroadenomes in non-affected breast | Infiltrating ductal Breast AC | II |
| **PATIENT 29** | Male | 77 | 1,80 | 63 | 19 | Hypertension, auricular fibrillation, chronic bronchitis | Multiple myeloma | |
| **PATIENT 30** | Female | 58 | 1,55 | 60 | 25 | subdural hematoma after fall | Multiple myeloma, leukemic | |

The chemotherapy treatment (anti-cancer drug and chemotherapy cycles) and the C-phycocyanin protocol of administration (treatment period, time of starting the treatment and dose of C-phycocyanin) are summarized in the following Table:

| Patient number | Chemotherapy treatment | | C-Phycocyanin Protocol Info | | | | |
|---|---|---|---|---|---|---|---|
| | Active ingredient | Cycles | Objective | C-Phycocyanin Starting Date | Treatment Period (months) | Start of C-Phycocyanin in Chemo treatment | C-Phycocyanin Dosage* (mg/kg/day) |
| **PATIENT 20** | TAXOL | 12 adjuvant | P | 29.07.2020 | 9 months | Before Chemo | 1.8 |
| **PATIENT 21** | TAXOL | 12 adjuvant | P | 16.07.2020 | 6 months | Before Chemo | 1 |
| **PATIENT 22** | Capecitabin | Neoadjuvant | P | 5.12.2020 | 14 months | After starting Chemo | 0.5 |
| **PATIENT 23** | FOLFOX | 12 adjuvant | P | 21.09.2020 | 10 months | Before Chemo | 1 |
| **PATIENT 24** | Taxol | 12 adjuvant | T | 11.12.2020 | 6 months | After C4 | 1 |
| **PATIENT 25** | Taxol Albumin | Neoadjuvant | T | 3.03.2020 | 3 weeks | At C5 | 2 |
| **PATIENT 26** | FOLFIRINO X | Neo-and /Adjuvant | T | 3.03.2021 | 3 months | | 4.5 |
| **PATIENT 27** | FOLFOX | Adjuvant | P | 11.03.2021 | 6 months | Before chemo | 1.2/2.4 D-1 |
| **PATIENT 28** | TAXOL | Adjuvant | P | 6.04.2021 | 6 months | Before chemo | 1.4/2.8 D-1 |
| **PATIENT 29** | Lenalidomide | | P | 20.01.2021 | 6 months | After starting Chemo | 1 |
| **PATIENT 30** | Bortezomib | | P | 20.02.2021 | 3 months | Before Chemo | 1 |

The degree of peripheral neuropathy (PN) and its location suffered by the patients before and after the administration of the corresponding protocol of C-phycocyanin is mentioned below:
P: prevention; T: treatment

| **Patient number** | **Neuropathy** | |
|---|---|---|
| | Neuropathy Grade (PN)/location **BEFORE** C-Phycocyanin | Neuropathy Grade (PN) **AFTER** C-Phycocyanin |
| **PATIENT 20** | **0** | **0** |
| **PATIENT 21** | **0** | **0** |
| **PATIENT 22** | **0** | **0** |
| **PATIENT 23** | **0** | **0** |
| **PATIENT 24** | **1 /toes** | **0** |
| **PATIENT 25** | **3 /legs** | **1/ toes** |
| **PATIENT 26** | **3 /legs and hands** | 0 |
| **PATIENT 27** | **0** | **0** |
| **PATIENT 28** | **0** | **0** |
| **PATIENT 29** | **0** | **0** |
| **PATIENT 30** | **0** | **0** |

**Conclusion**

The above disclosed results showed that the administration of an amount from 0.1 to 4.5 mg/Kg/day of C-phytocyanin according to the present invention is useful for the treatment or prevention of peripherical neuropathies induced by chemotherapy regardless the type of chemotherapeutical active ingredient used and the particular patient clinical conditions as such the type or grade of cancer diagnosticated.

Particularly, no neuropathic symptomatology has been observed in those patients submitted to chemotherapeutic treatment without prior neuropathic symptomatology. And totally or hardly totally reduction of the prior neuropathy acquired in previous chemotherapeutic treatments were also observed.

As it is shown in the experimental data, the treatment with an amount from 0.1 to 4.5 mg/Kg/day of C-phytocyanin can be started before the start of the chemotherapeutic treatment for the prevention of the appearance of neuropathy or for the treatment of prior acquired peripheric neuropathy. Furthermore, the C-phytocyanin can be also administered when the chemotherapeutic treatment has already been started, even during one chemotherapeutic treatment or at the end of the chemotherapeutic treatment. In the case of administration C-phycocyanin during the chemotherapeutic treatment, it is recommended the administration of a first dose the day before the start of the chemotherapeutic cycle, followed by a subsequent (second) administration after the ending of the chemotherapeutic treatment (cycle). Particularly, the day after which the anti-cancer drug is totally eliminated from the body. Thus, the first day of C-phycocyanin starts the day that corresponds to the double of the half-life of the anti-cancer drug.

To sum up, in the treatment assay, 27% of patients suffered from grade 1 CIPN, 7% grade 2, 60% grade 3 and 7% grade 4 before starting C-phycocyanin treatment. Nevertheless, after completion of the treatment with C-phycocyanin, 60% were cured (no CIPN symptomatology) and 40% had ONLY grade 1 of CIPN. Meanwhile, in the prevention assay, 78% of patients did not show any symptoms of CIPN two months after their chemotherapy regimens were finished and 22% of them had ONLY grade 1 CIPN.

**1.2.2. Treatment of infection induced- peripheral neuropathy**

**1.2.2.1. Herpes virus-induced peripheral neuropathy**

The utility of C-phycocyanin in the treatment of (herpes) virus-induced peripheral neuropathy is shown herein below. This data was compiled from patient treated by specialist during the period ranging from April 2020 and September 2021. The patient was diagnosed herpes zoster on the face and received antiviral treatment either before or during the treatment with C-phycocyanin.

The degree of peripheral neuropathy (PN) suffered by the patient was assessed by a specialist visiting the patients and assigned from PN0 to PN4 levels as mentioned above. The patient information, patient clinical conditions, antiviral treatment, C-phycocyanin protocol of administration, degree of peripheral neuropathy (PN) and its location suffered by the patients before and after the administration the appropriate amount (from 0.1 to 4.5mg/Kg/day) of C-phycocyanin according to the present invention is summarised herein below.

**Patient 32 (treatment)**

Patient data

Female, 70 years, 1.64 m height, 78 Kg weight, Body Mass Index 29
Prior conditions: sedentary, HTA, overweight, smoker 1 pack/day, basocellular epitheliome (2013), arrythmia,
Prior Herpes Type Diagnostic: left facial herpes zoster
Start Dermatone: 26.02.2021

Herpes treatment (PHN):

Days 60;
Treatments: Eliquis, flecainida, bisoprolol, espironolactone, zolpidem
Food Supplements: None

Phycocyanin Protocol:
Phycocyanin Starting Date: 23.04.2021
Duration of Treatment

Phycocyanin Starting Date: 23.04.2021
Duration of Treatment: 8 months
Phycocyanin Dosage (mg/kg/day): 0.5
Neuropathy grade before start of C-phycocyanin treatment: PN3 left face, eye
Neuropathy grade after C-phycocyanin treatment: PN0
Time to resolution of symptoms: 60 days
Time to resolution of neuropathy symptoms: 15days after start of the C-phycocyanin treatment

**1.2.2.2. Coronavirus-induced peripheral neuropathy**

The utility of C-phycocyanin in the treatment of coronavirus-induced peripheral neuropathy is shown herein below. This data was compiled from patient treated by specialist during the period ranging from April 2020 and September 2021. The patients were diagnosed with post Covid-19 peripheral neuropathy receiving at least treatment for the acute respiratory syndrome before and/or during the treatment with C-phycocyanin.

The degree of peripheral neuropathy (PN) suffered by the patient was assessed by a specialist visiting the patients and assigned from PN0 to PN4 levels as mentioned above. The patient information, patient clinical conditions, antiviral treatment, C-phycocyanin protocol of administration, degree of peripheral neuropathy (PN) and its location suffered by the patients before and after the administration the appropriate amount (from 0.1 to 4.5mg/Kg/day) of C-phycocyanin according to the present invention is summarised herein below.

**Patient 41 (treatment)**

Patient data

Female, 72 years, 1.62 m height, 63 Kg weight, Body Mass Index 24
Prior conditions: sedentary, severe asthenia PN, hospitalisation, induced coma, intubation, hypothyroidism and anaemia
Previous treatment: Eutirox, Vit C, Q10, pre/probiotics
Type Diagnostic: peripheral neuropathy post COVID-19

Days COVID PN: 30

Phycocyanin Protocol:

Phycocyanin Starting Date: 12.05.2021
Duration of phycocyanin Treatment: 14 days
Phycocyanin Dosage (mg/kg/day): 2.0
Neuropathy grade before start of C-phycocyanin treatment: PN3-4 legs and arms
Neuropathy grade after C-phycocyanin treatment: PN0
Time to resolution of neuropathy symptoms: 14 days after start of the C-phycocyanin treatment

**Patient 42 (treatment)**

Patient data

Male, 59 years, 1.81 m height, 115 Kg weight, Body Mass Index 35
Prior conditions: sedentary, severe asthenia PN, obesity, hospitalisation, induced coma, intubation
Previous treatment: Enoxaparin, inhaled salbutamol, paracetamol, Zn picolinate, Vit C, Vit D
Type Diagnostic: Peripheral neuropathy post COVID-19
Days COVID PN: 40

Phycocyanin Protocol:

Phycocyanin Starting Date: 28.04.2020
Duration of phycocyanin Treatment: 7 days
Phycocyanin Dosage (mg/kg/day): 0.7
Neuropathy grade before start of C-phycocyanin treatment: PN3-4 legs and arms
Neuropathy grade after C-phycocyanin treatment: PN0
Time to resolution of neuropathy symptoms: 7 days after start of the C-phycocyanin treatment

**Conclusion**

The above disclosed result showed that the administration of C-phytocyanin according to the present invention is also useful for the treatment of peripherical neuropathies induced by infections, particularly by virus such as Herpes virus and coronavirus virus in a short period of time after the start of the C-phycocyanin treatment.

**1.2.3. Treatment of Diabetes-induced peripheral neuropathy**

The utility of C-phycocyanin in the treatment of diabetes-induced peripheral neuropathy is shown herein below. The data was compiled from patients treated by specialist during the period ranging from January 2021 and September 2021. The patients were diagnosed diabetes type 2 and received antidiabetic oral treatment both before and during the treatment with C-phycocyanin.

The degree of peripheral neuropathy (PN) suffered by the patient was assessed by a specialist visiting the patients and assigned from PN0 to PN4 levels as mentioned above. The patient information, patient clinical conditions, the antidiabetic treatment, the C-phycocyanin protocol of administration, the degree of peripheral neuropathy (PN) and its location suffered by the patients before and after the administration of the corresponding protocol of C-phycocyanin is summarised herein below.

**Patient 33 (treatment)**

Patient data

Male, 58 years, 1.84 m height, 100 Kg weight, Body Mass Index 30
Prior conditions: sedentary, Hypertension arterial, renal insufficiency, dyslipidaemia, rheumatoid psoriasis, enolism, overweight, heart insufficiency, hepatitis, diabetes (non insulin dependent)
Glycemia: 158g/L; Glycated Hb: 7%
Prior pharmacological treatment: Dapagliflozin, sacubitril + valsartan, bisoprolol, sintrom, furosemide, plelerona.
Prior Food supplements: Magnesium and vitamin C.

C-Phycocyanin treatment data

Start of C-phycocyanin treatment: 01-Jan-2021
C-Phycocyanin treatment period: 9 months
C-Phycocyanin dosage: 1.2 mg/kg/day
Neuropathy grade before start of C-phycocyanin treatment: PN2 (in feet and legs)
Neuropathy grade after C-phycocyanin treatment: PN0
Time to resolution of neuropathy symptoms: 15days after start of the C-phycocyanin treatment

**Patient 34 (treatment)**

Patient data

Male, 72 years, 1.74m height, 98 Kg weight, Body Mass Index 32
Prior conditions: Hypertension arterial, dyslipidaemia, obesity arteritis (stent), diabetes (non insulin dependent)
Glycemia: 119g/L; Glycated Hb: 5.5%
Prior pharmacological treatment: Atorvastatin, clopidogrel, acetyl salicylate, pantoprazole, sitagliptin, metformin, gliclazide, hydrochlorothiazide, losartan
Prior Food supplements: Milk thistle + Dandelion

C-Phycocyanin treatment data

Start of C-phycocyanin treatment: 26-July-2021
C-Phycocyanin treatment period: 2 months
C-Phycocyanin dosage: 1 mg/kg/day
Neuropathy grade before start of C-phycocyanin treatment: PN4 (in feet and legs)
Neuropathy grade after C-phycocyanin treatment: PN2 (in feet and legs)

**Conclusion**

The above disclosed results showed that the administration of C-phytocyanin according to the present invention is also useful for the treatment of peripherical neuropathies induced by diabetes in a short period of time after the start of the C-phycocyanin treatment.

**1.3. Treatment and/or prevention of peripheral neuropathy by administration of crude (diluted) extract of *Arthrospira platensis***

In the following examples 35-40 C-phycocyanin was administered to patients suffering from or susceptible of suffering a peripheral neuropath in the form of an aqueous solution comprising the desired daily dose of C-phycocyanin according to the present invention.

The solution was prepared by dissolving of sufficient quantity of a product consisting of a mixture of the crude extract from *Arthrospira platensis* containing with a titrated content of C-phycocyanin of 40% calculated using the formula of Equation 1 reported above in mineral water just before oral administration to obtain an aqueous solution of approximately 8 mg of C-phycocyanin/ml of solution. As it is disclosed herein below, an effective amount from 0.1 to 0.6 mg of C-phytocyanin/kg/day was administered.

**1.3.1. Treatment and/or prevention of chemotherapy-induced peripheral neuropathy**

The utility of C-phycocyanin in the prevention of chemotherapy-induced peripheral neuropathy is shown in the following examples.

The data shown in examples 35-40 was compiled from patients treated by oncologists at different hospitals

during the period ranging from years 2015 to 2018. All patients had been diagnosed cancer by an oncologist and received chemotherapy either before or during the treatment with C-phycocyanin.

The degree of peripheral neuropathy (PN) suffered by the patient was assessed by an oncologist visiting the patients and assigned from PN0 to PN4 levels as mentioned above. And, the chemotherapeutical treatment details are the same mentioned in previous section for patients 1-18.

The patient information and patient clinical conditions are summarized in the following Table:

| **Patient Information** | | | | | | **Patient Clinical Condition** | | |
|---|---|---|---|---|---|---|---|---|
| **Patient number** | **Sex** | **Age** | **Height (m)** | **Weight (kg)** | **BMI** | **Prior Conditions** | **Cancer Diagnostic** | **Stage** |
| **PATIENT 35** | Female | 45 | 1,74 | 56 | 18 | pregnancy at 37 | Breast, ductal, triple negative pT2N0M0 | I |
| **PATIENT 36** | Female | 48 | 1,68 | 64 | 23 | leukaemia at 30 y.o., bone marrow transplant | cholangiocarc inoma, M1 PC | IV |
| **PATIENT 37** | Male | 68 | 1,64 | 78 | 29 | infantile poliomyelitis, cholecystectomy, right inguinal hernia, prostate cancer treated with decapeptyl, HTA, liver steatosis | Colon AC | IV |
| **PATIENT 38** | Male | 81 | 1,68 | 56 | 20 | HTA, hypertrophy benign de prostate, diabetes type II | Colon AC pT3N1M0 | III |
| **PATIENT 39** | Female | 53 | 1,62 | 69 | 26 | discal hernia, overweight | breast, ductal AC pT1cpN0M0 | I |
| **PATIENT 40** | Female | 56 | 1,69 | 59 | 21 | severe stress, ovary cysts | ovary, clear cells | III |

The chemotherapy treatment and the C-phycocyanin protocol of administration are summarized in the following Table:

| Patient number | Chemotherapy treatment | | C-Phycocyanin Protocol Info | | | | |
|---|---|---|---|---|---|---|---|
| | **Active ingredient** | **Cycles** | **Objective** | **C-Phycocyanin Starting Date** | **Treatment Period** | **Start of C-Phycocyanin in Chemo treatment** | **C-Phycocyanin Dosage* (mg/kg/day)** |
| **PATIENT 35** | AC + Paclitaxel adjuvant | 12 x Taxol | prevention | 25.06.2018 | 4 months | from C1 | **0.2** |
| **PATIENT 36** | Gemcitabine + Oxaliplatin | 13 adjuvant | prevention | 20.04.2017 | 10 months | from C1 | 0.2-0.6 |
| **PATIENT 37** | FOLFOX 4 | 12 adjuvant | prevention | 8.07.2016 | 2 years | from C1 | 0.2 |
| **PATIENT 38** | FOLFOX 4 | 12 adjuvant | prevention | 30.01.2015 | 18 months | from C1 | 0.2 |
| **PATIENT 39** | AC / Docetaxel | 4 docetaxel adjuvant | prevention | 15.01.2015 | 12 months | from C1 | 0.2 |
| **PATIENT 40** | Carbo Platine + Paclitaxel | 4x adjuvant | prevention | 9.02.2018 | 2 months | from C1 | **0.1** |
| Neoadjuvant: the chemotherapy is Before Surgery. Adjuvant: the chemotherapy is After Surgery | | | | | | | |

The degree of peripheral neuropathy (PN) and its location suffered by the patients before and after the administration of the corresponding protocol of C-phycocyanin is mentioned below:

| Patient number | Neuropathy | |
|---|---|---|
| | Neuropathy Grade (PN)/location **BEFORE** C-Phycocyanin | Neuropathy Grade (PN)/location **AFTER** C-Phycocyanin |
| **PATIENT 35** | 0 | **1/big toe** |
| **PATIENT 36** | 0 | 1 |
| **PATIENT 37** | 0 | 1 |
| **PATIENT 38** | 0 | 1 |
| **PATIENT 39** | 0 | 1 |
| **PATIENT 40** | 0 | 1 |

**Conclusion**

The above disclosed results showed that the administration of an amount from 0.1 to 4.5 mg/Kg/day of C-phytocyanin according to the present invention regardless the concentration of C-phycocyanin present in the extract of *Arthrospira platensis* used as starting material is useful for the treatment and/or prevention of peripherical neuropathies (particularly induced by chemotherapy). As it is mentioned above, the solution was prepared by dissolving of sufficient quantity of a product consisting of a mixture of the crude extract from *Arthrospira platensis* containing with a titrated content of C-phycocyanin of 40%. In fact, the administration of C-phycocyanin during the chemotherapeutic treatment was performed the day before the start of the chemotherapeutic cycle and followed by a subsequent (second) administration after the end of the chemotherapeutic cycle; particularly, the day after the anticancer drug has been totally eliminated from the body (corresponding to the double of the half-life of the anticancer drug).

As it is shown in the above results, the treatment with doses of C-phytocyanin about the lowest end point of the claimed range allows preventing the appearance of severe (grades PN2-4) peripherical neuropathy symptomatology, regardless the chemotherapeutic drug and the type/grade of cancer diagnosticated. in fact, all patients (patients 35-40) treated with C-phycocyanin according to the present invention develop peripheral neuropathy in a mild grade (grade 1). In fact, all patients treated only developed mild CIP at the end of the treatment after completing all cycles of chemotherapy. Meanwhile, as it is mentioned above, after the treatment with Paclitaxel, 75% of patients developed a peripheral neuropathy in grade 2-4. And the use of oxaliplatin triggers the appearance of peripheral neuropathy in grades 2-4 in 50-90% of the patients.

The invention is defined in claims 1-15. Herein described is the following subject-matter:

Clause 1. C-phycocyanin for use in the treatment or prevention of chemotherapy-induced peripheral neuropathy.

Clause 2. A composition comprising C-phycocyanin and at least one pharmaceutically acceptable excipient for use in the treatment or prevention of chemotherapy-induced peripheral neuropathy.

Clause 3. C-phycocyanin for use or composition for use according to any one of the preceding clauses wherein said C-phycocyanin is contained in an extract of phycobiliproteins obtained from cyanobacteria or microalgae.

Clause 4. C-phycocyanin for use or composition for use according to any one of the preceding clauses characterized in that the extract is obtained from *Spirulina Platensis.*

Clause 5. C-phycocyanin for use or composition for use according to any one of clauses 3 to 4 wherein the extract contains more than 80% by weight of C-phycocyanin, preferably more than 85 % weight of C-phycocyanin and most preferably more than 90 % weight of C-phycocyanin.

Clause 6. C-phycocyanin for use or composition for use according to any one of clauses 3 to 5 wherein a solution of 22,4 mg of the extract in 1000 ml of deionized water has UV-visible spectrum showing a ratio $A_{616}/A_{650}$ higher than 3, preferably higher than 3.5 and most preferably higher than 3.61, wherein $A_{616}$ is the absorbance at 616 nm and $A_{650}$, wherein $A_{650}$ is the absorbance at 650 nm.

Clause 7. C-phycocyanin for use or composition for use according to any one of the preceding clauses wherein the peripheral neuropathy has been induced by a taxane, a platinum compound, a vinca-alkaloid, or combinations thereof.

Clause 8. C-phycocyanin for use or composition for use according to clause 7 wherein the taxane is selected from the group consisting of paclitaxel, docetaxel, tesetaxel and cabazitalex, the platinum compound is selected from the group consisting of cisplatin, carboplatin and oxaliplatin and the vinca alkaloid is selected from the group consisting of vincristine and vinblastine.

Clause 9. C-phycocyanin for use or composition for use according to any one of the preceding clauses wherein said composition is administered orally.

Clause 10. C-phycocyanin for use or composition for use according to any one of the preceding clauses wherein the treatment and or the prevention are carried out by administering to a subject in need thereof an amount of C-phycocyanin or of the composition comprising C-phycocyanin equivalent to 0.1 to 4 mg of C-phycocyanin per kilogram of weight of the patient for one or more days.

Clause 11. C-phycocyanin for use or composition for use according to clause 10 wherein C-phycocyanin or the composition comprising C-phycocyanin are administered during a period overlapping completely or partially with the period during which the patient is receiving chemotherapeutic treatment and wherein said C-phycocyanin or said composition comprising C-phycocyanin are administered on days in which the patient does not receive any dose of the chemotherapeutic treatment.

Clause 12. Composition for use according to any preceding clauses wherein said C-phycocyanin or said composition are used for the treatment of chemotherapy-induced peripheral neuropathy and administered after the start of administration of the chemotherapy.

## Claims

1. A product which is C-phycocyanin, or an extract comprising C-phycocyanin, for use in the treatment and/or prevention of a peripheral neuropathy, wherein the treatment and/or prevention comprises administering C-phycocyanin in an amount from 0.1 to 4.5 mg per Kilogram of body weight per day.

2. A composition comprising the product as defined in claim 1 and one or more pharmaceutically acceptable excipients or carriers, for use in the treatment and/or prevention of a peripheral neuropathy; wherein the treatment and/or prevention comprises administering C-phycocyanin in an amount from 0.1 to 4.5 mg per Kilogram of body weight per day.

3. The product for use according to claim 1; or alternatively, the composition for use according to claim 2, wherein the treatment and/or prevention comprises administering C-phycocyanin in an amount from 0.5 to 4.5 mg per Kilogram of body weight per day.

4. The product for use according to any of the claims 1 or 3; or alternatively, the composition for use according to any of the claims 2 or 3, wherein the treatment and/or prevention comprises administering C-phycocyanin in an amount from 1 to 4.5 mg per Kilogram of body weight per day.

5. The product for use according to any of the claims 1, 3 or 4; or alternatively, the composition for use according to any of the claims 2-4, wherein:

   said product is an extract comprising C-phycocyanin that further comprises at least another phycobiliprotein;
   or alternatively, said product is an extract comprising C-phycocyanin obtained from cyanobacteria;
   or alternatively, said product is an extract comprising C-phycocyanin that further comprises at least another phycobiliprotein and is obtained from cyanobacteria.

6. The product for use according to any of the claims 1, or 3-5; or alternatively, the composition for use according to any one of the claims 2-5, wherein the product is an extract comprising C-phycocyanin obtained from *Spirulina Platensis.*

**7.** The product for use according to any of the claims 1, or 3-6; or alternatively, the composition for use, according to any of the claims 2-6, wherein the product is an extract comprising C-phycocyanin in an amount equal to or more than 40% by weight of C-phycocyanin; preferably in an amount equal to or more than 60% by weight of C-phycocyanin; preferably equal to or more than 80% by weight of C-phycocyanin; preferably equal to or more than 85 % by weight of C-phycocyanin; and preferably equal to or more than 90 % by weight of C-phycocyanin.

**8.** The composition for use according to any of the claims 2-7, which is a composition selected from the group consisting of injectable composition and oral composition; particularly the composition is an oral composition selected from the group consisting of a liquid oral composition and a solid oral composition.

**9.** The composition for use according to claim 8, wherein the composition is a liquid composition in form of a solution; particularly, the composition is a liquid composition in form of a solution comprising 22,4 mg of the extract comprising more than 90% by weight of C-phycocyanin in 1000 ml of deionized water showing in the UV-visible spectrum a ratio $A_{616}/A_{650}$ higher than 3; preferably higher than 3.5; and preferably higher than 3.61, wherein $A_{616}$ is the absorbance at 616 nm and $A_{650}$ is the absorbance at 650 nm.

**10.** The product for use according to any of the claims 1, or 3-7; or alternatively, the composition for use according to any of the claims 2-9, wherein the peripheral neuropathy is selected from the group consisting of a chemotherapy induced peripheral neuropathy, radiotherapy induced peripheral neuropathy, infection induced peripheral neuropathy, diabetes induced peripheral neuropathy, atherosclerosis induced peripheral neuropathy, chronic alcohol consumption peripheral neuropathy, lung injury induced peripheral neuropathy, kidney injury induced peripheral neuropathy, hepatic injury induced peripheral neuropathy and a combination thereof.

**11.** The product for use according to any of the claims 1, 3-7, or 10; or alternatively, the composition for use according to any of the claims 2-10, wherein the peripheral neuropathy is a chemotherapy induced peripheral neuropathy.

**12.** The product for use according to any of the claims 1, 3-7, or 10-11; or alternatively, the composition for use according to any of the claims 2-11, wherein: the peripheral neuropathy is a chemotherapy induced peripheral neuropathy, and the chemotherapy comprises administering one or more anti-cancer agents selected form the group consisting of taxane-based anti-cancer agent; a platinum-based anti-cancer agent; a vinca-alkaloid anti-cancer agent; bortezomib; thalidomide and analogs thereof; alkylating anticancer agents; and a combination thereof;
Particularly, the chemotherapy comprises administering one or more anti-cancer agents selected form the group consisting of taxane-based anti-cancer agent; a platinum-based anti-cancer agent; a vinca-alkaloid anti-cancer agent; and a combination thereof.

**13.** The product for use according to any of the claims 1, 3-7, or 10-12; or alternatively, the composition for use according to any of the claims 2-12, wherein the peripheral neuropathy is a chemotherapy induced peripheral neuropathy, and the chemotherapy comprises administering one or more anti-cancer agents selected form the group consisting of paclitaxel, docetaxel, tesetaxel, cabazitalex, cisplatin, carboplatin, oxaliplatin, vincristine and vinblastine; bortezomib, thalidomide, lenalilomide, and combination thereof.

**14.** The product for use according to any of the claims 1, 3-7, or 10-13; or alternatively, the composition for use according to any of the claims 2-13, wherein the peripheral neuropathy is a chemotherapy induced peripheral neuropathy and the treatment and/or prevention comprises administering C-phycocyanin during a period overlapping completely or partially with the period during which the patient is receiving chemotherapeutic treatment;
particularly the treatment and/or prevention comprises administering the C-phycocyanin on days in which the patient does not receive any dose of the chemotherapeutic treatment.

**15.** The product for use according to any of the claims 1, 3-7, or 10-14; or alternatively, the composition for use, according to any of the claims 2-14, wherein the treatment and/or prevention comprises:

administering the C-phycocyanin before the start of administration of the chemotherapy;
or alternatively, administering the C-phycocyanin after the start of administration of the chemotherapy; particularly, after the end of administration of the chemotherapy;
or alternatively, administering firstly, the C-phycocyanin before the start of administration of the chemotherapy; and administering secondly, the C-phycocyanin after the start of administration of the chemotherapy; particularly, after the end of administration of the chemotherapy.

**Patentansprüche**

1. Ein Produkt, das C-Phycocyanin, oder ein Extrakt, der C-Phycocyanin umfasst, ist zur Verwendung bei der Behandlung und/oder Prävention einer peripheren Neuropathie, wobei die Behandlung und/oder die Prävention die Verabreichung von C-Phycocyanin in einer Menge von 0,1 bis 4,5 mg pro Kilogramm Körpergewicht pro Tag umfasst.

2. Eine Zusammensetzung umfassend das Produkt wie in Anspruch 1 definiert und einen oder mehrere pharmazeutisch akzeptable Hilfsstoffe oder Trägerstoffe zur Verwendung bei der Behandlung und/oder Prävention einer peripheren Neuropathie; wobei die Behandlung und/oder Prävention die Verabreichung von C-Phycocyanin in einer Menge von 0,1 bis 4,5 mg pro Kilogramm Körpergewicht pro Tag umfasst.

3. Das Produkt zur Verwendung nach Anspruch 1; oder ersatzweise die Zusammensetzung zur Verwendung nach Anspruch 2, wobei die Behandlung und/oder die Prävention die Verabreichung von C-Phycocyanin in einer Menge von 0,5 bis 4,5 mg pro Kilogramm Körpergewicht pro Tag umfasst.

4. Das Produkt zur Verwendung nach einem der Ansprüche 1 oder 3; oder ersatzweise die Zusammensetzung zur Verwendung nach einem der Ansprüche 2 oder 3, wobei die Behandlung und/oder die Prävention die Verabreichung von C-Phycocyanin in einer Menge von 1 bis 4,5 mg pro Kilogramm Körpergewicht pro Tag umfasst.

5. Das Produkt zur Verwendung nach einem der Ansprüche 1, 3 oder 4; oder ersatzweise die Zusammensetzung zur Verwendung nach einem der Ansprüche 2 bis 4, wobei:

   das Produkt ein C-Phycocyanin enthaltender Extrakt ist, der ferner mindestens ein weiteres Phycobiliprotein enthält;
   oder, ersatzweise, das Produkt ein C-Phycocyanin enthaltender Extrakt ist, der aus Cyanobakterien gewonnen wird;
   oder, ersatzweise, das Produkt ein C-Phycocyanin enthaltender Extrakt ist, der ferner mindestens ein weiteres Phycobiliprotein umfasst und aus Cyanobakterien gewonnen wird.

6. Das Produkt zur Verwendung nach einem der Ansprüche 1 oder 3 bis 5; oder ersatzweise die Zusammensetzung zur Verwendung nach einem der Ansprüche 2 bis 5, wobei das Produkt ein Extrakt ist, der C-Phycocyanin umfasst, das aus *Spirulina Platensis* gewonnen wird.

7. Das Produkt zur Verwendung nach einem der Ansprüche 1 oder 3 bis 6; oder ersatzweise die Zusammensetzung zur Verwendung nach einem der Ansprüche 2 bis 6, wobei das Produkt ein Extrakt ist, der C-Phycocyanin in einer Menge von gleich oder mehr als 40 Gew.- % C-Phycocyanin umfasst; vorzugsweise in einer Menge von gleich oder mehr als 60 Gew.- % C-Phycocyanin; vorzugsweise von gleich oder mehr als 80 Gew.- % C-Phycocyanin; vorzugsweise von gleich oder mehr als 85 Gew.- % C-Phycocyanin; und vorzugsweise von gleich oder mehr als 90 Gew.- % C-Phycocyanin.

8. Die Zusammensetzung zur Verwendung nach einem der Ansprüche 2 bis 7, welche eine Zusammensetzung ist, die aus der Gruppe ausgewählt ist, die aus einer injizierbaren Zusammensetzung und einer oralen Zusammensetzung besteht; insbesondere ist die Zusammensetzung eine orale Zusammensetzung, die aus der Gruppe ausgewählt ist, die aus einer flüssigen oralen Zusammensetzung und einer festen oralen Zusammensetzung besteht.

9. Die Zusammensetzung zur Verwendung nach Anspruch 8, wobei die Zusammensetzung eine flüssige Zusammensetzung in Form einer Lösung ist; insbesondere ist die Zusammensetzung eine flüssige Zusammensetzung in Form einer Lösung, die 22,4 mg des Extrakts umfasst, der mehr als 90 Gew.- % C-Phycocyanin in 1000 ml entionisiertem Wasser umfasst, die im UV-sichtbaren Spektrum ein Verhältnis $A_{616}/A_{650}$ von mehr als 3; vorzugsweise von mehr als 3,5; und vorzugsweise von mehr als 3,61 zeigt, wobei $A_{616}$ die Absorption bei 616 nm und $A_{650}$ die Absorption bei 650 nm ist.

10. Das Produkt zur Verwendung nach einem der Ansprüche 1 oder 3 bis 7; oder ersatzweise die Zusammensetzung zur Verwendung nach einem der Ansprüche 2 bis 9, wobei die periphere Neuropathie ausgewählt ist aus der Gruppe bestehend aus einer durch Chemotherapie induzierten peripheren Neuropathie, einer durch Strahlentherapie induzierten peripheren Neuropathie, einer durch Infektion induzierten peripheren Neuropathie, einer durch Diabetes induzierten peripheren Neuropathie, einer durch Atherosklerose induzierten peripheren Neuropathie, einer durch chronischen Alkoholkonsum induzierten peripheren Neuropathie, einer durch eine Lungenverletzung induzierten

peripheren Neuropathie, einer durch eine Nierenverletzung induzierten peripheren Neuropathie, einer durch eine Leberverletzung induzierten peripheren Neuropathie und einer Kombination davon.

11. Das Produkt zur Verwendung nach einem der Ansprüche 1, 3 bis 7 oder 10; oder ersatzweise die Zusammensetzung zur Verwendung nach einem der Ansprüche 2 bis 10, wobei die periphere Neuropathie eine durch Chemotherapie induzierte periphere Neuropathie ist.

12. Das Produkt zur Verwendung nach einem der Ansprüche 1, 3 bis 7 oder 10 bis 11; oder ersatzweise die Zusammensetzung zur Verwendung nach einem der Ansprüche 2 bis 11, wobei: die periphere Neuropathie eine durch Chemotherapie induzierte periphere Neuropathie ist und die Chemotherapie die Verabreichung von einem oder mehreren Antikrebsmitteln umfasst, die aus der Gruppe ausgewählt sind, die aus einem Antikrebsmittel auf Taxanbasis; einem Antikrebsmittel auf Platinbasis; einem Vincaalkaloid-Antikrebsmittel; Bortezomib; Thalidomid und Analogen davon; alkylierenden Antikrebsmitteln; und einer Kombination davon besteht;
Insbesondere umfasst die Chemotherapie die Verabreichung von einem oder mehreren Antikrebsmitteln, die ausgewählt sind aus der Gruppe bestehend aus einem Antikrebsmittel auf Taxanbasis; einem Antikrebsmittel auf Platinbasis; einem Vincaalkaloid-Antikrebsmittel; und einer Kombination davon.

13. Das Produkt zur Verwendung nach einem der Ansprüche 1, 3 bis 7 oder 10 bis 12; oder ersatzweise die Zusammensetzung zur Verwendung nach einem der Ansprüche 2 bis 12, wobei die periphere Neuropathie eine durch Chemotherapie induzierte periphere Neuropathie ist und die Chemotherapie die Verabreichung von einem oder mehreren Antikrebsmitteln umfasst, die aus der Gruppe ausgewählt sind, die aus Paclitaxel, Docetaxel, Tesetaxel, Cabazitalex, Cisplatin, Carboplatin, Oxaliplatin, Vincristin und Vinblastin; Bortezomib, Thalidomid, Lenalilomid und einer Kombination davon besteht.

14. Das Produkt zur Verwendung nach einem der Ansprüche 1, 3 bis 7 oder 10 bis 13; oder ersatzweise die Zusammensetzung zur Verwendung nach einem der Ansprüche 2 bis 13, wobei die periphere Neuropathie eine durch Chemotherapie induzierte periphere Neuropathie ist und die Behandlung und/oder die Prävention die Verabreichung von C-Phycocyanin während eines Zeitraums umfasst, der sich vollständig oder teilweise mit dem Zeitraum überschneidet, während dessen der Patient eine chemotherapeutische Behandlung erhält;
insbesondere umfasst die Behandlung und/oder die Prävention die Verabreichung des C-Phycocyanins an Tagen, an denen der Patient keine Dosis der chemotherapeutischen Behandlung erhält.

15. Das Produkt zur Verwendung nach einem der Ansprüche 1, 3 bis 7 oder 10 bis 14; oder ersatzweise die Zusammensetzung zur Verwendung nach einem der Ansprüche 2 bis 14, wobei die Behandlung und/oder die Prävention Folgendes umfasst:

Verabreichung des C-Phycocyanins vor Beginn der Verabreichung der Chemotherapie;
oder ersatzweise die Verabreichung des C-Phycocyanins nach Beginn der Verabreichung der Chemotherapie; insbesondere nach Ende der Verabreichung der Chemotherapie;
oder ersatzweise die Verabreichung des C-Phycocyanins erstens vor Beginn der Verabreichung der Chemotherapie; und die Verabreichung des C-Phycocyanins zweitens nach Beginn der Verabreichung der Chemotherapie; insbesondere nach Ende der Verabreichung der Chemotherapie.

## Revendications

1. Un produit qui est la C-phycocyanine, ou un extrait comprenant de la C-phycocyanine, destiné à être utilisé dans le traitement et/ou la prévention d'une neuropathie périphérique, dans lequel le traitement et/ou la prévention comprend l'administration de C-phycocyanine en une quantité de 0,1 à 4,5 mg par kilogramme de poids corporel par jour.

2. Une composition comprenant le produit tel que défini dans la revendication 1 et un ou plusieurs excipients ou véhicules pharmaceutiquement acceptables, pour une utilisation dans le traitement et/ou la prévention d'une neuropathie périphérique ; dans laquelle le traitement et/ou la prévention comprend l'administration de C-phycocyanine en une quantité de 0,1 à 4,5 mg par kilogramme de poids corporel par jour.

3. Le produit pour l'utilisation selon la revendication 1 ; ou en variante, la composition pour l'utilisation selon la revendication 2, dans lequel le traitement et/ou la prévention comprend l'administration de C-phycocyanine en une quantité de 0,5 à 4,5 mg par kilogramme de poids corporel par jour.

4. Le produit pour l'utilisation selon l'une quelconque des revendications 1 ou 3 ; ou en variante, la composition pour l'utilisation selon l'une quelconque des revendications 2 ou 3, dans lequel le traitement et/ou la prévention comprend l'administration de C-phycocyanine en une quantité de 1 à 4,5 mg par kilogramme de poids corporel par jour.

5. Le produit pour l'utilisation selon l'une quelconque des revendications 1, 3 ou 4 ; ou en variante, la composition pour l'utilisation selon l'une quelconque des revendications 2 à 4, où :

ledit produit est un extrait comprenant de la C-phycocyanine qui comprend en outre au moins une autre phycobiliprotéine ;
ou en variante, ledit produit est un extrait comprenant de la C-phycocyanine obtenue à partir de cyanobactéries ;
ou en variante, ledit produit est un extrait comprenant de la C-phycocyanine qui comprend en outre au moins une autre phycobiliprotéine et est obtenu à partir de cyanobactéries.

6. Le produit pour l'utilisation selon l'une quelconque des revendications 1 ou 3 à 5 ; ou en variante, la composition pour l'utilisation selon l'une quelconque des revendications 2 à 5, où le produit est un extrait comprenant de la C-phycocyanine obtenue à partir de *Spirulina Platensis.*

7. Le produit pour l'utilisation selon l'une quelconque des revendications 1 ou 3 à 6 ; ou en variante, la composition pour l'utilisation, selon l'une quelconque des revendications 2 à 6, dans lequel le produit est un extrait comprenant de la C-phycocyanine en une quantité égale ou supérieure à 40 % en poids de C-phycocyanine ; de préférence en une quantité égale ou supérieure à 60 % en poids de C-phycocyanine ; de préférence égale ou supérieure à 80 % en poids de C-phycocyanine ; de préférence égale ou supérieure à 85 % en poids de C-phycocyanine ; et de préférence égale ou supérieure à 90 % en poids de C-phycocyanine.

8. La composition pour l'utilisation selon l'une quelconque des revendications 2 à 7, qui est une composition choisie dans le groupe constitué par une composition injectable et une composition orale ; en particulier, la composition est une composition orale choisie dans le groupe constitué par une composition orale liquide et une composition orale solide.

9. La composition pour l'utilisation selon la revendication 8, dans laquelle la composition est une composition liquide sous la forme d'une solution ; en particulier, la composition est une composition liquide sous la forme d'une solution comprenant 22,4 mg de l'extrait comprenant plus de 90 % en poids de C-phycocyanine dans 1000 ml d'eau désionisée présentant dans le spectre UV-visible un rapport $A_{616}/A_{650}$ supérieur à 3 ; de préférence supérieur à 3,5 ; et de préférence supérieur à 3,61, dans lequel $A_{616}$ est l'absorbance à 616 nm et $A_{650}$ est l'absorbance à 650 nm.

10. Le produit à utiliser selon l'une quelconque des revendications 1 ou 3 à 7 ; ou en variante, la composition à utiliser selon l'une quelconque des revendications 2 à 9, où la neuropathie périphérique est choisie dans le groupe constitué par une neuropathie périphérique induite par la chimiothérapie, une neuropathie périphérique induite par la radiothérapie, une neuropathie périphérique induite par une infection, une neuropathie périphérique induite par le diabète, une neuropathie périphérique induite par l'athérosclérose, une neuropathie périphérique due à la consommation d'alcool chronique, une neuropathie périphérique induite par une lésion pulmonaire, une neuropathie périphérique induite par une lésion rénale, une neuropathie périphérique induite par une lésion hépatique et une combinaison de celles-ci.

11. Le produit pour l'utilisation selon l'une quelconque des revendications 1, 3 à 7 ou 10 ; ou en variante, la composition pour l'utilisation selon l'une quelconque des revendications 2 à 10, dans laquelle la neuropathie périphérique est une neuropathie périphérique induite par la chimiothérapie.

12. Le produit pour l'utilisation selon l'une quelconque des revendications 1, 3 à 7 ou 10 à 11 ; ou en variante, la composition pour l'utilisation selon l'une quelconque des revendications 2 à 11, où : la neuropathie périphérique est une neuropathie périphérique induite par une chimiothérapie, et la chimiothérapie comprend l'administration d'un ou plusieurs agents anticancéreux choisis dans le groupe constitué par un agent anticancéreux à base de taxane ; un agent anticancéreux à base de platine ; un agent anticancéreux vinca-alcaloïde ; le bortézomib ; le thalidomide et ses analogues ; des agents anticancéreux alkylants ; et une combinaison de ceux-ci ;
En particulier, la chimiothérapie comprend l'administration d'un ou de plusieurs agents anticancéreux choisis dans le groupe constitué par un agent anticancéreux à base de taxane ; un agent anticancéreux à base de platine ; un agent anticancéreux vinca-alcaloïde ; et une combinaison de ceux-ci.

**13.** Le produit pour l'utilisation selon l'une quelconque des revendications 1, 3 à 7 ou 10 à 12 ; ou en variante, la composition pour l'utilisation selon l'une quelconque des revendications 2 à 12, dans laquelle la neuropathie périphérique est une neuropathie périphérique induite par la chimiothérapie, et la chimiothérapie comprend l'administration d'un ou plusieurs agents anticancéreux choisis dans le groupe constitué par le paclitaxel, le docétaxel, le tesétaxel, le cabazitalex, le cisplatine, le carboplatine, l'oxaliplatine, la vincristine et la vinblastine ; le bortézomib, le thalidomide, le lénalilomide et une combinaison de ceux-ci.

**14.** Le produit pour l'utilisation selon l'une quelconque des revendications 1, 3 à 7 ou 10 à 13 ; ou en variante, la composition pour l'utilisation selon l'une quelconque des revendications 2 à 13, dans laquelle la neuropathie périphérique est une neuropathie périphérique induite par la chimiothérapie et le traitement et/ou la prévention comprend l'administration de C-phycocyanine pendant une période chevauchant complètement ou partiellement la période pendant laquelle le patient reçoit un traitement chimiothérapeutique ;
en particulier le traitement et/ou la prévention comprend l'administration de la C-phycocyanine les jours dans lesquels le patient ne reçoit aucune dose du traitement chimiothérapeutique.

**15.** Le produit pour l'utilisation selon l'une quelconque des revendications 1, 3 à 7 ou 10 à 14 ; ou en variante, la composition pour l'utilisation, selon l'une quelconque des revendications 2 à 14, dans lequel le traitement et/ou la prévention comprend :

administrer la C-phycocyanine avant le début de l'administration de la chimiothérapie ;
ou en variante, l'administration de la C-phycocyanine après le début de l'administration de la chimiothérapie ; en particulier, après la fin de l'administration de la chimiothérapie ;
ou en variante, l'administration, d'une part, de la C-phycocyanine avant le début de l'administration de la chimiothérapie ; et l'administration, d'autre part, de la C-phycocyanine après le début de l'administration de la chimiothérapie ; en particulier, après la fin de l'administration de la chimiothérapie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 20382835
- US 2014287021 A
- EP 3192504 A
- US 201258180 B


### Non-patent literature cited in the description


- **T. ARMSTRONG et al.** *Oncol Nurs Forum,* 2005, vol. 32, 305-311
- **C. VISOVSKY et al.** *Clin J Oncol Nurs,* 2008, vol. 12, 243-247
- **A. BHAGRA ; R.D. RAO.** *Curr Oncol Rep,* 2007, vol. 9, 290-299
- **G. CAVALETTI et al.** *Curr Treat Options Neurol,* 2011, vol. 13, 180-190
- **SCHLOSS J.M. et al.** *Clin Nutr,* 2013, vol. 32 (6), 888-893
- **HERSHMAN D.L. et al.** *J Clin Oncol,* 2014, vol. 32 (18), 1941-1967
- **BRYANT D.A. et al.** *Journal of General Microbiology,* 1982, vol. 128, 835-844
- **M. RITO-PALOMARES ; L. NUNEZ ; D. AMADOR.** *J. Chem. Technol. Biotechnol.,* 2001, vol. 76, 1273
- **FIGUEIRA et al.** *Brazilian Journal of Chemical Engineering,* vol. 35 (3), 1117-1128
- **BENNET.** *J. Cell Biol.,* 1973, vol. 58, 419-435
- **A. VONSHAK.** Spirulina platensis (Arthrospira). Taylor & Francis, 1997, 175-204
- **KEYS A. et al.** Indices of relative weight and obesity. *International Journal of Epidemiology,* 2014, vol. 43 (3), 655-665